(19) Europäisches Patentamt
European Patent Office
Office européen des brevets

(11) EP 2 826 865 B1

(12) EUROPEAN PATENT SPECIFICATION

(45) Date of publication and mention of the grant of the patent:
**21.06.2017 Bulletin 2017/25**

(51) Int Cl.:
*C12Q 1/68* (2006.01)      *G06F 19/22* (2011.01)

(21) Application number: **12865785.5**

(22) Date of filing: **20.01.2012**

(86) International application number:
**PCT/CN2012/070680**

(87) International publication number:
**WO 2013/107048 (25.07.2013 Gazette 2013/30)**

(54) **METHOD AND SYSTEM FOR DETERMINING WHETHER COPY NUMBER VARIATION EXISTS IN SAMPLE GENOME, AND COMPUTER READABLE MEDIUM**

VERFAHREN UND SYSTEM ZUR BESTIMMUNG DER EXISTENZ EINER KOPIEZAHLVARIANTE IN EINER GENOMPROBE UND COMPUTERLESBARES MEDIUM

PROCÉDÉ ET SYSTÈME POUR DÉTERMINER S'IL EXISTE UNE VARIABILITÉ DU NOMBRE DE COPIES DANS UN GÉNOME ÉCHANTILLON, ET SUPPORT LISIBLE PAR ORDINATEUR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.01.2015 Bulletin 2015/04**

(73) Proprietor: **BGI Diagnosis Co., Ltd.**
**Guangdong 518083 (CN)**

(72) Inventors:
• **YIN, Xuyang**
  **Shenzhen**
  **Guangdong 518083 (CN)**
• **ZHANG, Chunlei**
  **Shenzhen**
  **Guangdong 518083 (CN)**
• **CHEN, Shengpei**
  **Shenzhen**
  **Guangdong 518083 (CN)**
• **ZHANG, Chunsheng**
  **Shenzhen**
  **Guangdong 518083 (CN)**
• **PAN, Xiaoyu**
  **Shenzhen**
  **Guangdong 518083 (CN)**
• **JIANG, Hui**
  **Shenzhen**
  **Guangdong 518083 (CN)**
• **ZHANG, Xiuqing**
  **Shenzhen**
  **Guangdong 518083 (CN)**

(74) Representative: **Huenges, Martin**
**Maiwald Patentanwalts GmbH**
**Elisenhof**
**Elisenstrasse 3**
**80335 München (DE)**

(56) References cited:
**EP-A1- 2 772 549      WO-A1-2011/091046**
**WO-A2-2007/129000      WO-A2-2007/131135**
**WO-A2-2010/033578      WO-A2-2012/006291**
**US-A1- 2003 082 606**

• **ALEX S NORD ET AL: "Accurate and exact CNV identification from targeted high-throughput sequence data", BMC GENOMICS, BIOMED CENTRAL LTD, LONDON, UK, vol. 12, no. 1, 12 April 2011 (2011-04-12), page 184, XP021097730, ISSN: 1471-2164, DOI: 10.1186/1471-2164-12-184**
• **DEREK Y CHIANG ET AL: "High-resolution mapping of copy-number alterations with massively parallel sequencing", NATURE METHODS, vol. 6, no. 1, 30 November 2008 (2008-11-30), pages 99-103, XP055065796, ISSN: 1548-7091, DOI: 10.1038/nmeth.1276**
• **S. YOON ET AL: "Sensitive and accurate detection of copy number variants using read depth of coverage", GENOME RESEARCH, vol. 19, no. 9, 5 August 2009 (2009-08-05), pages 1586-1592, XP055167321, ISSN: 1088-9051, DOI: 10.1101/gr.092981.109**

EP 2 826 865 B1

- **T. CHU ET AL: "Statistical model for whole genome sequencing and its application to minimally invasive diagnosis of fetal genetic disease", BIOINFORMATICS, vol. 25, no. 10, 23 March 2009 (2009-03-23), pages 1244-1250, XP055018601, ISSN: 1367-4803, DOI: 10.1093/bioinformatics/btp156**

- **CHUNLEI ZHANG ET AL: "A Single Cell Level Based Method for Copy Number Variation Analysis by Low Coverage Massively Parallel Sequencing", PLOS ONE, vol. 8, no. 1, 23 January 2013 (2013-01-23), page e54236, XP55136784, ISSN: 1932-6203, DOI: 10.1371/journal.pone.0054236**

**Description**

TECHNICAL FIELD

Embodiments of the present disclosure generally relate to a method of determining whether copy number variation presents in a genome sample, and a system and a computer readable medium thereof.

BACKGROUND

In fields of scientific research and application, problems of analyzing a single cell, a plurality of cells, or a trace of nucleic acid sample usually come out, for example, Pre-implantation Genetic Diagnosis (PGD) and Pre-implantation Genetic Screening (PGS) in a field of assisted reproductive technology involve analysis with a single germ cell, a single blastomeric cell or an embryonic cell; a field of non-invasive prenatal diagnosis technology involves problem of detecting a trace of fetal cells in maternal peripheral blood; Metagenomics involves analysis with a single or a trace of biological cell in environment; and disease or physical research involves analysis with a single cell in tissue or body fluid. Yoon et al. (Genome Research 2009, 19(9):1586-1592) discloses a method for determining copy number variants based on read depth coverage and provides what is termed an "event wise testing" method based on significance testing. However, currently the method of determining copy number variation still needs to be improved.

SUMMARY

Embodiments of the present disclosure seek to solve at least one of the problems existing in prior art to at least some extent. The scope of the present invention is defined in appended claims 1 to 14. Embodiments of a first broad aspect of the present disclosure provide a method of determining whether copy number variation presents in a genome sample. According to embodiments of the present disclosure, the method may comprise following steps: sequencing the genome sample, to obtain a sequencing result consisting of a plurality of reads; aligning the sequencing result to a reference genome sequence, to determine a distribution of the reads in the reference genome sequence; determining a plurality of breakpoints in the reference genome sequence based on the distribution of the reads in the reference genome sequence, wherein the number of reads has significance at both sides of the breakpoints; determining a detection window in the reference genome based on the plurality of the breakpoints; determining a first parameter based on reads falling in the detection window; and determining whether the copy number variation presents in the genome sample against the detection window based on difference between the first parameter and a preset threshold. By using the method of determining whether copy number variation presents in a genome sample according to embodiments of the present disclosure, whether copy number variation presents in a genome sample may be effectively determined, which is suitable for various copy number variations, included but not limited to aneuploidy of chromosome, deletion of chromosome, and addition, micro-deletion and micro-repetition of chromosome fragments.

Embodiments of a second broad aspect of the present disclosure provide a system for determining whether copy number variation presents in a genome sample. According to embodiments of the present disclosure, the system may comprise:

a sequencing apparatus, configured to sequence the genome sample, to obtain a sequencing result consisting of a plurality of reads; an analysis apparatus, connected to the sequencing apparatus, configured to determine whether copy number variation presents in the genome sample based on the sequencing result, wherein the analysis apparatus further comprises: an aligning unit, configured to align the sequencing result to a reference genome sequence, to determine a distribution of the reads in the reference genome sequence; a breakpoint determining unit, connected to the aligning unit, configured to determine a plurality of breakpoints in the reference genome sequence, based on the distribution of the reads in the reference genome sequence, wherein the number of reads has significance at both sides of the breakpoints; a detection window determining unit, connected to the breakpoint determining unit, configured to determine a detection window in the reference genome based on the plurality of the breakpoints; a parameter determining unit, connected to the detection window determining unit, configured to determine a first parameter based on reads falling in the detection window; and a determining unit, connected to the parameter determining unit, configured to determine whether the copy number variation presents in the genome sample against the detection window based on difference between the first parameter and a preset threshold. By using the system for determining whether copy number variation presents in a genome sample according to embodiments of the present disclosure, the method of determining whether copy number variation presents in a genome sample according to embodiments of the present disclosure may be effectively implemented, which is suitable for various copy number variations, included but not limited to aneuploidy of chromosome, deletion of chromosome, and addition, micro-deletion and micro-repetition of chromosome fragments.

Embodiments of a third broad aspect of the present disclosure provide a computer readable medium. According to embodiments of the present disclosure, the computer readable medium is configured to perform by a processer to determine whether copy number variation presents in a genome sample through following steps: aligning the sequencing result to a reference genome sequence, to determine a distribution of the reads in the reference genome sequence; determining a plurality of breakpoints in the reference genome sequence based on the distribution of the reads in the reference genome sequence, wherein the number of reads has significance at both sides of the breakpoints; determining a detection window in the reference genome based on the plurality of the breakpoints; determining a first parameter based on reads falling in the detection window; and determining whether the copy number variation presents in the genome sample against the detection window based on difference between the first parameter and a preset threshold. By virtue of the computer readable medium, the method of determining whether copy number variation presents in a genome sample according to embodiments of the present disclosure may be effectively implemented, so as to effectively determine whether copy number variation presents in a genome sample, which is suitable for various copy number variations, included but not limited to aneuploidy of chromosome, deletion of chromosome, and addition, micro-deletion and micro-repetition of chromosome fragments.

Additional aspects and advantages of embodiments of present disclosure will be given in part in the following descriptions, become apparent in part from the following descriptions, or be learned from the practice of the embodiments of the present disclosure.

BRIEF DESCRIPTION OF THE DRAWINGS

These and other aspects and advantages of embodiments of the present disclosure will become apparent and more readily appreciated from the following descriptions made with reference the accompanying drawings, in which:

Fig.1 is a flow chart showing a method of determining whether copy number variation presents in a genome sample according to an embodiment of the present disclosure;

Fig.2 is a schematic diagram showing a system for whether copy number variation presents in a genome sample according to an embodiment of the present disclosure;

Fig.3 is a flow chart showing a method of determining whether copy number variation presents in a genome sample according to another embodiment of the present disclosure;

Fig.4 is an image showing chromosome karyotype analysis of a sample S1 according to embodiments of the present disclosure, in which the left panel shows a result obtained by the method of detecting copy number variation according to an embodiment of the present disclosure with a single embryonic cell which has been subjected to a whole genome amplification, the right panel shows a result obtained by directly sequencing (without subjecting to the whole genome amplification firstly) with DNA extracted from the same single embryonic cell; and

Fig.5 an image showing chromosome karyotype analysis of a sample S2 according to embodiments of the present disclosure, in which the left panel shows a result obtained by the method of detecting copy number variation according to an embodiment of the present disclosure with a single embryonic cell which has been subjected to a whole genome amplification, the right panel shows a result obtained by directly sequencing (without subjecting to the whole genome amplification firstly) with DNA extracted from the same single embryonic cell.

DETAILED DESCRIPTION

Reference will be made in detail to embodiments of the present disclosure. The same or similar elements and the elements having same or similar functions are denoted by like reference numerals throughout the descriptions. The embodiments described herein with reference to drawings are explanatory, illustrative, and used to generally understand the present disclosure. The embodiments shall not be construed to limit the present disclosure

In addition, terms such as "first" and "second" are used herein for purposes of description and are not intended to indicate or imply relative importance or significance. Thus, features defined with "first" or "second" may explicitly or implicitly include one or more of said feature. Furthermore, in descriptions of the present disclosure, unless otherwise specified, "a plurality of" refers to two or more. If not specified, in formula or signs used herein, the same alphabet represents a same meaning.

**I. Method of determining whether copy number variation presents in a genome sample**

According to a first aspect of the present disclosure, there is provided a method of determining whether copy number variation presents in a genome sample in the present disclosure. Term of "copy number variation (CNV)" used herein refers to abnormality of chromosome or chromosome fragment copy number, including but not limited to chromosome aneuploidy, chromosome fragment deletion, addition, micro-deletion and micro-repeat of chromosome fragment.

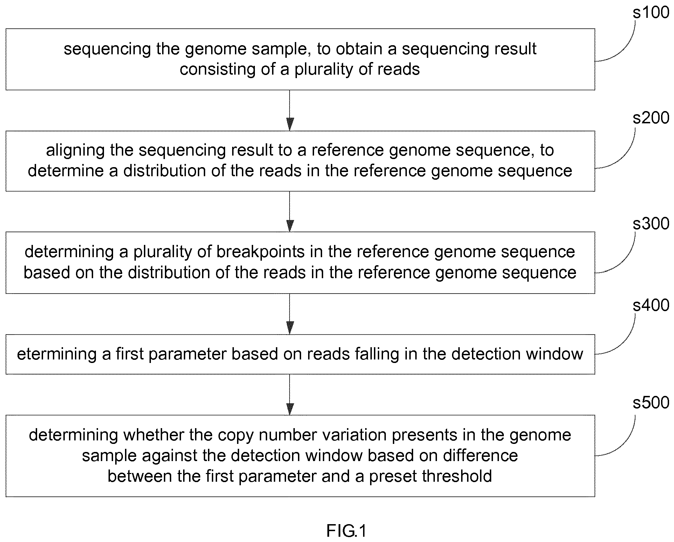

Referring to Fig.1, the method of determining whether copy number variation presents in a genome sample according to embodiments of the present disclosure comprises:

## S100: sequencing the genome sample, to obtain a sequencing result consisting of a plurality of reads

According to embodiments of the present disclosure, types of the genome samples with which the method of the present disclosure are not subjected to special restrictions, which may be a whole genome or a part of a genome, for example, chromosome or chromosome fragment. Besides, according to embodiments of the present disclosure, prior to the step of sequencing the genome sample, the method of determining whether copy number variation presents in a genome sample may further comprise a step of extracting the genome sample from a biological sample. Accordingly, the biological sample may be directly used as raw material, for obtaining information regarding whether the biological sample has copy number variation, so as to reflect health status of organisms. According to embodiments of the present disclosure, the used biological sample is not subjected to special restrictions. According to some specific examples of the present disclosure, the biological sample is any one selected from a group consisting of blood, urine, saliva, tissue, germ cells, oosperm, blastomere and embryo. It would be appreciated by those skilled in the art that different biological samples may be used for analyzing different diseases. Accordingly, these samples may be conveniently obtained from organisms, and different samples may be used specifically directing to certain diseases, so as to selecting specific means for analyzing the certain diseases. For example, for a subject possibly suffering a certain cancer, a sample may be collected from cancerous tissue or juxtacancerous tissue, from which cells are further isolated for analysis, accordingly, whether such tissue become cancerous may be accurately determined as early as possible. According a specific example of the present disclosure, a single cell may be used as the biological sample. According to embodiments of the present disclosure, methods and devices of isolating a single cell from a biological sample are not subjected to special restrictions. According to some specific examples of the present disclosure, a single cell may be isolated from a biological sample using at least one of dilution, mouth-controlled pipette, micromanipulation (micro-dissection is preferred), flow cytometry isolation, microfluidics. Accordingly, a single cell may be effectively and conveniently obtained from a biological sample, to implement subsequent steps. Then, efficiency of determining whether copy number variation presents in a genome sample may be further improved.

Besides, according to embodiments of the present disclosure, methods of sequencing the genome sample are not subjected to special restrictions. According to an embodiment of the present disclosure, the step of sequencing the genome sample further comprises following sub-steps of: firstly amplifying the genome sample, to obtain an amplified genome sample; secondly, constructing a sequencing-library with the amplified genome sample; finally sequencing the constructed sequencing-library, to obtain the sequencing result consisting of a plurality of reads. Accordingly, whole genome information of the sequencing result of the genome sample may be effectively obtained, and a single cell genome or a trace of nucleic acid sample may be subjected to effective sequencing, which may further improve efficiency of determining whether copy number variation presents in a genome sample. Those skilled in the art may choose different methods of constructing a sequencing-library in accordance with specific solutions used in genome sequencing techniques. A detailed process of constructing a genome sequencing-library may refer to a specification provided by sequencing-instrument manufacturer, such as Illumina Company, for example Multiplexing Sample Preparation Guide (Part#1005063; Feb 2010).

Optionally, for the step of extracting the genome sample from the biological sample when being a single cell, according to embodiments of the present disclosure, the method may further comprise a step of lysing the single cell, to release whole genome of the single cell. According to some examples of the present disclosure, methods of lysing the single cell to release whole genome are not subjected to special restrictions, as long as the single cell is lysed, preferably the single cell is fully lysed. According to specific examples of the present disclosure, the single cell is lysed using an alkaline lysate, to release whole genome of the single cell. Inventors of the present disclosure find out that the step of lysing the single cell may effectively lyse the single cell to release whole genome, and accuracy may be improved when subjecting the released whole genome to sequencing, which may further improve efficiency of determining whether copy number variation presents in a genome sample. According to embodiments of the present disclosure, methods of amplifying a single cell whole genome are not subjected to special restrictions, a PCR-based method may be used, for example PEP-PCR, DOP-PCR and OmniPlex WGA; a non-PCR-based method may be also used, for example Multiple Displacement Amplification (MDA). According to specific examples of the present disclosure, the PCR-based method is preferably used, for example OmniPlex WGA. A commercial kit, including but not limited to GenomePlex from Sigma Aldrich, PicoPlex from Rubicon Genomics, REPLI-g from Qiagen, illustra GenomiPhi from GE Healthcare and etc, may be used. According to specific examples of the present disclosure, prior to the sub-step of constructing a sequencing-library, the single cell whole genome may be amplified by OmniPlex WGA. Accordingly, the whole genome may be effectively amplified, which may further improve efficiency of determining whether copy number variation presents in a genome sample. According to embodiments of the present disclosure, the sub-step of sequencing the whole genome sequencing-library is performed by at least one selected from Next-Generation sequencing technology consisting of Hiseq system of Illumina Company, Miseq system of Illumina Company, Genome Analyzer (GA) system of Illumina Company, 454 FLX of Roche Company, SOLiD system of Applied Biosystems Company, Ion Torrent system of Life Technologie Company. Accordingly, characteristics of high-throughput and deep sequencing of these sequencing apparatus may be used, which further improves efficiency of determining whether copy number variation presents in a genome sample. Obviously, it would be appreciated by those skilled in the art that other sequencing methods and apparatuses may also be used for whole genome sequencing, for example Third-Generation sequencing technology (i.e., single molecule sequencing technology) such as any one of HeliScope system from Helicos BioSciences Company, RS system from PacBio Company and etc., as well as more advanced sequencing technology which may be developed later. According to embodiments of the present disclosure, lengths of sequencing data obtained by whole genome sequencing are not subjected to special restrictions. According to a specific example of the present disclosure, the plurality of sequencing data have an average length of about 50 bp. The inventors of the present disclosure surprisingly find out that sequencing data having a length of about 50 bp may greatly faciliate subjecting the sequencing data to analyzing, which improves analysis efficiency and significantly reduces cost for analysis, by which further improves efficiency of determining chromosome aneuploidy of a single cell and reduces cost of determining chromosome aneuploidy of a single cell. Term of "average length" used herein refers to a mean value of length values of every sequencing data.

**S200: aligning the sequencing result to a reference genome sequence, to determine a distribution of the reads in the reference genome sequence**

After completing the step of sequencing the genome sample, the obtained sequencing result includes a plurality of sequencing data. The obtained sequencing result is aligned to a reference genome sequence, so as to determine a location of the obtained sequencing result in the reference genome sequence. According to embodiments of the present disclosure, any known methods may be used to calculate the total number of these sequencing data. For example, software provided by sequencing instrument manufacturer may be used for analysis. Short Oligonucleotide Analysis Package (SOAP) and Burrows-Wheeler Aligner (BWA) are preferably used, which align reads to a reference genome sequence, to obtain a location of reads in the reference sequence. A default parameter provided by program of the software may be used in alignment, or a parameter may be selected by those skilled in the art as required. In an embodiment of the present disclosure, SOAPaligner/soap2 is used as alignment software.

According to embodiments of the present disclosure, the reference genome sequence may be a standard human genome reference sequence in NCBI database (for example may be hg18, NCBI Build 36); or may be a part of a known genome sequence, for example may be at least one sequence selected from a group consisting of human chromosome 21, chromosome 18, chromosome 13, chromosome X and chromosome Y.

According to embodiments of the present disclosure, by the step of aligning the sequencing result to a reference genome sequence, sequences which are uniquely aligned to the reference genome sequence may be selected for subsequent analysis. Accordingly, interference to analysis of copy number variation by repeat sequences may be avoided, which further improves efficiency of determining whether copy number variation presents in a genome sample.

**S300: determining a plurality of breakpoints in the reference genome sequence based on the distribution of the reads in the reference genome sequence**

Term of "breakpoints" used herein refers to such kind of sites in a genome, in which the number of the reads on either side of the site are significantly different between these two regions. As reads derive from the genome sample, when a certain region presents copy number variation in the genome sample, the number of the reads corresponded in the region also changes significantly. Accordingly, after determining a plurality of breakpoints, copy number variation probably presents in a region between two successive breakpoints may be preliminary determined.

According to embodiments of the present disclosure, the step of determining a plurality of breakpoints in the reference genome sequence further comprises following sub-steps:

Firstly, the reference genome sequence is divided into a plurality of primary windows having a predetermined length, and reads falling in each of the plurality of primary windows are determined. According to specific examples of the present disclosure, by conventional alignment programs, reads contained in the obtained sequencing result may be aligned to the reference genome sequence, by which reads falling in each of the plurality of primary windows may be determined, for example, it may be accomplished in the step S200 above-described. According to specific examples of the present disclosure, the reads falling in each of the plurality of primary windows are uniquely-aligned reads. Accordingly, interference to analysis of copy number variation by repeat sequences may be avoided, which further improves efficiency of determining whether copy number variation presents in a genome sample.

Secondly, for at least one site in the reference genome sequence, determining the number of reads falling in the same number of the plurality of primary windows at both sides of the site. According to embodiments of the present disclosure, correlation analysis may be performed with all sites in the reference genome sequence, or with interested chromosome, for example such correlation analysis is performed with all sites in at least one of human chromosome 21, chromosome 18, chromosome 13, chromosome X and chromosome Y. According to embodiments of the present disclosure, each of the primary windows may have same or different length; an overlap may present between primary windows, as long as information of each primary window is known; each of the primary windows is preferably has a same length. According to embodiments of the present disclosure, each of plurality of the primary windows may have a length of 100 to 200 Kbp, preferably 150 Kbp. According to embodiments of the present disclosure, the number of the primary windows located at both sides of the site is not subjected to special restrictions, according to a specific example of the present disclosure, 100 of the primary windows may be selected from either side of the site respectively.

Thirdly, by statistical analysis, p value of the site may be determined, in which the p value represents that the number of reads falling in either side of the site has significance. If the p value of the site is smaller than a final p value, that the site is the breakpoints is determined. According to embodiments of the present disclosure, a range of the final p value may be determined by subjecting a known sequence sample to parallel analysis, according to a specific example of the present disclosure, the final p value is $1.1 \times 10^{-50}$.

According to an embodiment of the present disclosure, the sub-step of determining p value of the site further comprises:

For the selected site, primary windows having the same number at either side of the site are selected, the relative number of reads falling in each primary window $R_i$ is calculated, in which i represents the No. of the primary windows, the relative number of reads falling in all primary windows $R_i$ are subjected to Run-Test, to determine the p value of the site, in which
the relative number of reads is determined by following formula:

$$ R_i = \log_2 \left( \frac{r_i}{\overline{r}} \right) $$

in which $r_i$ represents the number of reads falling in the i-th primary window,

$$ \overline{r} = \frac{1}{n} \sum_{i=1}^{n} r_i \text{ ,} $$

n represents the total number of the primary windows.

In details, the step of subjecting all of the relative numbers of the reads falling in each of the plurality of primary windows to Run-Test further comprises: subjecting the relative number of reads falling in each of the plurality of primary windows Ri to a correction of GC content, to obtain corrected relative number of reads $\tilde{R}_i$; determining the normalized number of reads falling in each of the plurality of primary windows Zi based on the corrected relative number of reads; and subjecting all of the normalized numbers of reads falling in each of the plurality of primary windows Zi to Run-Test.
More specifically, the corrected relative number of reads $\tilde{R}_i$ is obtained by following steps:

Firstly GC content of each primary window is calculated;
Secondly, the GC content is divided into a plurality of regions in accordance with a predetermined value, and a mean value $M_s$ of the relative number of reads falling in each of the plurality of regions is calculated, in which s is No. of the plurality of regions, according to embodiments of the present disclosure, the predetermined value may be any numerical value in a range of 0.0005 to 0.01, of which a corresponding region has a length of 50 k to 300 k, 0.001 is preferred, by which may performing a correlation with an optimal power.
Thirdly, the corrected relative number of reads $\tilde{R}_i$ is determined based on the following formula: $\tilde{R}_i = R_i - M_s$.

Lastly, the normalized number of reads $Z_i$ is determined based on the following formula, in which

$$Z_i = \left( R_i - \tilde{R}_i - mean \right) / SD$$

$$mean = \frac{1}{n} \sum_{i=1}^{n} \left( R_i - \tilde{R}_i \right)$$

$$SD = \sqrt{\frac{1}{n-1} \sum_{i=1}^{n} \left( R_i - \tilde{R}_i - mean \right)^2}$$

.

Accordingly, the number of reads may be subjected to correlation by GC content. Thus, an interference caused by bias of genome amplification may be eliminated, by which improves accuracy and efficiency of determining whether copy number variation presents in a genome sample.

After the plurality of breakpoints has been determined, a possibility that copy number variation presents in a region between two successive breakpoints may be preliminary determined. Accordingly such regions may be taken as the detection windows for further determining whether copy number variation presents. In the case of obtaining relative more breakpoints in the preliminary determination, the obtained breakpoints may be subjected to further screening. Accordingly, according to embodiments of the present disclosure, the step of determining a detection window in the reference genome based on the plurality of the breakpoints further comprises:

> 1) determining a plurality of candidate breakpoints, wherein other breakpoints present both before and after the candidate breakpoints;
> 2) determining p value of each candidate breakpoint, and removing a candidate breakpoint having the final p value;
> 3) performing the step 2) with rest of the candidate breakpoints until all p values of the rest of the candidate breakpoints are smaller than the final p value, wherein the rest of the candidate breakpoints are taken as screened candidate breakpoints; and
> 4) determining a region between two successive screened candidate breakpoints as the detection window.

According to embodiments of the present disclosure, the p value of the candidate breakpoint is obtained by following steps:

> selecting a region between the candidate breakpoint and previous candidate breakpoint as a first candidate region, and selecting a region between the candidate breakpoint and next candidate breakpoint as a second candidate region; subjecting the normalized number of reads falling in the primary windows Zi which are included both in the first candidate region and the second candidate region to Run-Test (The Run-Test is a nonparametric test, evaluating significant difference between two populations using evenly distributed status of mixed elements with two populations. Details regarding such test may refer to Wald A. WJ. On a Test Whether Two Samples are from the Same Population. The Annals of Mathematical Statistics 1940; 11:147-162), to determine the p value of the candidate breakpoints.

According to embodiments of the present disclosure, the final p value is obtained by following steps:

> based on a sequencing result of a control sample, repeating the step of determining a detection window in the reference genome, and recording p values of the breakpoints which are removed each time until the number of the breakpoints is zero, in which term of "control sample" used herein refers to a sample of which copy number variation does not present in a known nucleotide sequence; and
> based on a distribution of the p values of removed breakpoints, the final p value is determined, for example, a distribution diagram is plotted with the p values of removed breakpoints, a p value having a maximal changing trend is taken as the final p value ($p_{final}$).

According to specific examples of the present disclosure, the final p value is $1.1 \times 10^{-50}$.

**S400: determining a first parameter based on reads falling in the detection window**

After the detection windows have been determined, reads contained in the detection windows may be subjected to statistical analysis, so as to determine whether copy number variation presents in the detection windows. According to an embodiments of the present disclosure, the step of determining the first parameter based on reads falling in the detection windows further comprises: determining a mean value among all of the normalized numbers of reads falling in each of the plurality of primary windows $\bar{Z}$ which are included in the detection windows, in which the mean value of the normalized number of reads $\bar{Z}$ is taken as the first parameter. The normalized number of reads has been specifically described above, which is omitted herein for brevity.

**S500: determining whether the copy number variation presents in the genome sample against the detection window based on difference between the first parameter and a preset threshold**

According to embodiments of the present disclosure, the determined first parameter may be compared with a preset threshold, then based on difference between the first parameter and the present threshold, whether the copy number variation presents in the genome sample is determined regarding the specific detection window. Based on the sequencing result of the genome sample, the number of reads falling in a certain window is positively related to content of the certain window in chromosome or genome, accordingly by subjecting reads deriving from a certain window in the sequencing result to statistical analysis, whether the copy number variation presents in the genome sample may be effectively determined based on the certain window. Term of "preset threshold" used herein refers to relative parameter based on the certain window obtained by repeating the operations and analysis in the above embodiments using a normal genome sample having a known sequence. It would be appreciated that, relative parameter based on the certain window and relative parameter of normal cells may be obtained by same sequencing conditions and mathematics methods. Here, the relative parameter of normal cells may be used as the preset threshold. Besides, term "preset" used herein should be broadly understood, which may be predetermined by experiment, or may be obtained by parallel experiments when analyzing the biological sample. Term "parallel experiment" should be broadly understood, which may refer to sequencing and analyzing unknown and known samples at the same time, or may refer to performing the steps of sequencing and analyzing under same conditions successively. According to embodiments of the present disclosure, the preset threshold comprises a first threshold and a second threshold, by comparing the first parameter $\bar{Z}$ to the first threshold and the second threshold, in the case of the first parameter $\bar{Z}$ smaller than the first threshold, copy number reducing is determined (i.e., deletion), in the case of the first parameter $\bar{Z}$ greater than the second threshold, copy number increasing is determined (i.e., addition), accordingly which type of the copy number variation may be determined. According to specific examples of the present disclosure, $\alpha = 0.05$ is set as a boundary of significance, by which type of the copy number variation is further determined.

By the method of determining whether copy number variation presents in a genome sample according to embodiments of the present disclosure, whether copy number variation in the genome sample may be effectively determined, which is suitable for various variations, including but not limited to chromosome aneuploidy, chromosome fragment deletion, fragment addition, addition, micro-deletion and micro-repeat of chromosome fragment. Copy number variation is the major factor inducing birth defect, which is also very common in embryo cultured *in vitro,* being a major reason leading to failure of reproduction *in vitro.* Copy number variation is also a pathogenic factor to many diseases such as cancer. The whole genome amplification is technique which can perform amplification in a range of whole genome with a single cell, a plurality cells or a trace of nucleic acid sample, which may increase sample amount on the premise of maintaining representativeness of the whole genome, to achieve the required sample amount. However, in general, a problem of amplification bias presents in the whole genome amplification, which brings in deviation to subsequent analysis. The method of determining whether copy number variation presents in a genome sample according to embodiments of the present disclosure, after the single cell or a trace of nucleic acid sample has been subjected to whole genome amplification, data is obtained by sequencing technology for analysis of copy number variation. On one hand, a problem of having difficulties in analyzing with a single cell or a trace of nucleic acid sample is solved by the whole genome amplification, on the other hand, bias to analyzing copy number variation induced by the whole genome amplification is avoided, which makes detection more accurate and more comprehensive, particularly detection efficiency may be further improved by a correlation of GC content. Besides, according to embodiments, during the sub-step of constructing sequencing-library with different samples, different indexes are introduced, by which a plurality samples may be subjected to test at the same time, which further improves efficiency of determining whether copy number variation presents in a genome sample. Using the method of determining whether copy number variation presents in a genome sample according to embodiments of the present disclosure, screening and diagnosing copy number variation prior to embryo implantation or noninvasive screening of fetal copy number variation may be determined, which is benefit to provide genetic counseling and basis for clinic decision; prenatal diagnosis may effectively prevent implantation of embryo with lesion, to present newborns with defects.

II System for determining whether copy number variation presents in a genome sample

According to a second aspect of the present disclosure, there is provided a system for determining whether copy number variation presents in a genome sample. Using the system may effectively implement the method of determining whether copy number variation presents in a genome sample above-described, so as to effectively determine whether copy number variation presents in the genome sample.

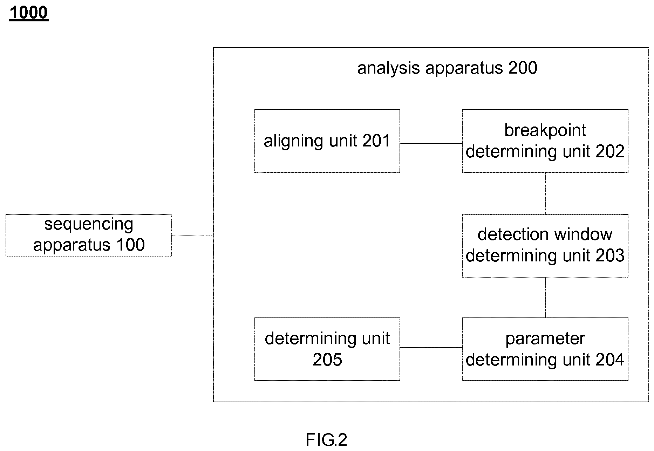

Referring to Fig.2, according to embodiments of the present disclosure, the system 1000 for determining whether copy number variation presents in a genome sample comprises: a sequencing apparatus 100 and an analysis apparatus 200.

According to embodiments of the present disclosure, the sequencing apparatus 100 is configured to sequence the genome sample, to obtain a sequencing result consisting of a plurality of reads. According to embodiments of the present disclosure, the system 1000 for determining whether copy number variation presents in a genome sample may further comprise a genome extracting apparatus (not shown in Figs). The genome extracting apparatus is configured to extract the genome sample from a biological sample, and the genome extracting apparatus is connected to the sequencing apparatus 100 for providing the genome sample. Accordingly, the biological sample may be directly used as raw material, to obtain information whether copy number variation presents in the biological sample, so as to reflect health status of organisms. According to embodiments of the present disclosure, the sequencing apparatus 100 may further comprise: a genome amplifying unit, a sequencing-library constructing unit and a sequencing unit, in which the genome amplifying unit is configured to amplify the genome sample; the sequencing-library constructing unit, connected to the genome amplifying unit, is configured to construct a sequencing-library with the amplified genome sample; and the sequencing unit, connected to the sequencing-library constructing unit, is configured to sequence the sequencing-library. According to embodiments of the present disclosure, the sub-step of sequencing the whole genome sequencing-library is performed by at least one selected from Next-Generation sequencing technology (such as Hiseq system of Illumina Company, Miseq system of Illumina Company, Genome Analyzer (GA) system of Illumina Company, 454 FLX of Roche Company, SOLiD system of Applied Biosystems Company, Ion Torrent system of Life Technologie Company) and single molecule sequencing apparatus. Accordingly, characteristics of high-throughput and deep sequencing of these sequencing apparatus may be used, which further improves efficiency of determining whether copy number variation presents in a genome sample.

According to embodiments of the present disclosure, the analysis apparatus 200 is connected to the sequencing apparatus 100, to determine whether copy number variation presents in a genome sample based on the sequencing result. According to embodiments of the present disclosure, the analysis apparatus 200 further comprises: an aligning unit 201, a breakpoint determining unit 202, a detection window determining unit 203, a parameter determining unit 204 and a determining unit 205, in which the aligning unit 201 is configured to align the sequencing result to a reference genome sequence, to determine a distribution of the reads in the reference genome sequence. According to embodiments of the present disclosure, a known human genome sequence is preserved in the aligning unit 201 as the reference genome sequence, optionally, the reference genome sequence is at least one selected from human chromosome 21, chromosome 18, chromosome 13, chromosome X and chromosome Y. The breakpoint determining unit 202, connected to the aligning unit 201, is configured to determine a plurality of breakpoints in the reference genome sequence, based on the distribution of the reads in the reference genome sequence, as described above, the number of reads has significance between two sides of the breakpoints. The detection window determining unit 203, connected to the breakpoint determining unit 202, is configured to determine a detection window in the reference genome based on the plurality of the breakpoints. The parameter determining unit 204, connected to the detection window determining unit, is configured to determine a first parameter based on reads falling in the detection window. The determining unit 205, connected to the parameter determining unit 204, configured to determine whether the copy number variation presents in the genome sample against the detection window based on difference between the first parameter and a preset threshold.

According to embodiments, the breakpoint determining unit 202 further comprises a module for performing following sub-steps:

dividing the reference genome sequence into a plurality of primary windows having a predetermined length, and determining reads falling in each of the plurality of the primary windows;

Firstly, the reference genome sequence is divided into a plurality of primary windows having a predetermined length, and reads falling in each of the plurality of primary windows are determined. According to specific examples of the present disclosure, by conventional alignment programs, reads contained in the obtained sequencing result may be aligned to the reference genome sequence, by which reads falling in each of the plurality of primary windows may be determined. According to embodiments of the present disclosure, each of the primary windows may have same or different length; an overlap may present between primary windows, as long as information of each primary window is known; each of the primary windows is preferably has a same length. According to embodiments of the present disclosure, each of the plurality of primary windows may have a length of 100 to 200 Kbp, preferably 150 Kbp. According to embodiments of the present disclosure, the number of the primary windows located at both sides of the site is not subjected to special restrictions, according to a specific example of the present disclosure, 100 of the primary windows may be selected from either side of the site respectively.

Secondly, p value of the site is determined; such p value may reflect significant difference of the number of reads between two sides of the site. Besides, the p value of the site is smaller than a final p value, the site is determined as the breakpoints. According to embodiments of the present disclosure, a range of the final p value may be determined by subjecting a known sequence sample to parallel analysis, according to a specific example of the present disclosure, the final p value is $1.1 \times 10^{-50}$.

According to embodiments of the present disclosure, the breakpoint determining unit 202 further comprises a module for performing following sub-steps:

For the selected site, the same number of the primary windows at either side of the site is selected respectively, and the relative number of reads falling in every primary window Ri is calculated, in which i represents No. of the primary windows,

the relative number of reads falling in all primary windows $R_i$ is subjected to Run-Test, o determine the p value of the site, in which

the relative number of reads is determined by following formula:

$$R_i = \log_2\left(\frac{r_i}{\bar{r}}\right)$$

in which $r_i$ represents the number of reads falling in the i-th primary window,

$$\bar{r} = \frac{1}{n}\sum_{i=1}^{n} r_i \, ,$$

n represents the total number of the primary windows.

According to embodiments of the present disclosure, the breakpoint determining unit 202 further comprises a module for performing followings to subject all of the relative numbers of the reads falling in each of the plurality of primary windows to Run-Test:

subjecting the relative number of reads falling in each of the plurality of primary windows $R_i$ to a correction of GC content, to obtain corrected relative number of reads $\tilde{R}_i$;

determining the normalized number of reads falling in each of the plurality of primary windows $Z_i$ based on the corrected relative number of reads; and

subjecting all of the normalized number of reads falling in each of the plurality of primary windows $Z_i$ to Run-Test.

According to embodiments of the present disclosure, the corrected relative number of reads $\tilde{R}_i$ is obtained by a module for performing following steps:

calculating GC content of each of the plurality of primary windows;

dividing the GC content into a plurality of regions in a unit of 0.001, and calculating a mean value $M_s$ among all of the relative numbers of reads falling in each of the plurality of regions, wherein s is No. of the plurality of regions;

determining the corrected relative number of reads $\tilde{R}_i$ based on the following formula:

$$\tilde{R}_i = R_i - M_s \, ;$$

determining the normalized number of reads $Z_i$ based on the following formula: wherein

$$Z_i = \left( R_i - \tilde{R}_i - mean \right) \Big/ SD \, ,$$

wherein

$$mean = \frac{1}{n} \sum_{i=1}^{n} \left( R_i - \tilde{R}_i \right) \, ,$$

$$SD = \sqrt{ \frac{1}{n-1} \sum_{i=1}^{n} \left( R_i - \tilde{R}_i - mean \right)^2 } \, .$$

After the plurality of breakpoints has been determined, a possibility that copy number variation presents in a region between two successive breakpoints may be preliminary determined. Accordingly such regions may be taken as the detection windows for further determining whether copy number variation presents. In the case of obtaining relative more breakpoints in the preliminary determination, the obtained breakpoints may be subjected to further screening. According to embodiments of the present disclosure, based on the plurality of the breakpoints, the detection window determining unit further comprises a module for performing followings:

1) determining a plurality of candidate breakpoints, wherein other breakpoints present both before and after the candidate breakpoints;
2) determining p value of each candidate breakpoint, and removing a candidate breakpoint having the final p value;
3) performing the step 2) with rest of the candidate breakpoints until p values of the rest of the candidate breakpoints all smaller than the final p value, wherein the rest of the candidate breakpoints are taken as screened candidate breakpoints; and
4) determining a region between two successive screened candidate breakpoints as the detection window.

In which, according to embodiments of the present disclosure, the p value of the candidate breakpoint is obtained by following steps:

selecting a region between the candidate breakpoint and previous candidate breakpoint as a first candidate region, and selecting a region between the candidate breakpoint and next candidate breakpoint as a second candidate region; subjecting the normalized number of reads falling in the primary windows Zi which are included both in the first candidate region and the second candidate region to Run-Test, to determine the p value of the candidate breakpoints.

According to embodiments of the present disclosure, the final p value is obtained by following steps:

based on a sequencing result of a control sample, repeating the step of determining a detection window in the reference genome, and recording p values of the breakpoints which are removed each time until the number of the breakpoints is zero; and
determining the final p value, based on a distribution of the p values of removed breakpoints, for example, a distribution diagram is plotted with the p values of removed breakpoints, a p value having a maximal changing trend is taken as the final p value ($p_{final}$).

According to specific examples of the present disclosure, the final p value is $1.1 \times 10^{-50}$.
According to embodiments of the present disclosure, the parameter determining unit 204 further comprises a module for performing followings: determining a mean value among all of the normalized numbers of reads falling in each of the plurality of primary windows $\overline{Z}$ which are included in the detection windows, in which the mean value of the normalized number of reads $\overline{Z}$ is taken as the first parameter. Furthermore, a preset threshold is preserved in the determining unit 205, accordingly, the determining unit 205 may compare the first parameter determined in the parameter determining unit 204, so as to determine whether copy number variation presents in the obtained detection windows, in which according to embodiments of the present disclosure, the preset threshold comprises: a first threshold and a second threshold, by comparing the first parameter $\overline{Z}$ to the first threshold and the second threshold, in the case of the first parameter $\overline{Z}$ smaller than the first threshold, copy number reducing is determined (i.e., deletion), in the case of the first parameter $\overline{Z}$ greater than the second threshold, copy number increasing is determined (i.e., addition), accordingly which type of the copy number variation may be determined. According to specific examples of the present disclosure, $\alpha$ =

0.05 is set as a boundary of significance, by which type of the copy number variation is further determined.

Accordingly, using the system for determining whether copy number variation presents in a genome sample according to embodiments of the present disclosure, the method of determining whether copy number variation presents in a genome sample according to embodiments of the present disclosure may be effectively implemented, so as to effectively determine whether copy number variation presents in the genome sample, which is suitable for various copy number variations, included but not limited to aneuploidy of chromosome, deletion of chromosome, and addition, micro-deletion and micro-repetition of chromosome fragments.

It should note that, it would be appreciated by those skilled in the art that, the above-described characteristics and advantages of the method of determining whether copy number variation presents in a genome sample is also suitable to the system for whether copy number variation presents in a genome sample, which are omitted for convenience and brevity.

### III. Computer readable medium

According to a third aspect of the present disclosure, there is provided a computer readable medium. According to embodiments of the present disclosure, an order is preserved in the computer readable medium, the order is configured to perform by a processer to determine whether copy number variation presents in a genome sample through following steps: aligning the sequencing result to a reference genome sequence, to determine a distribution of the reads in the reference genome sequence; determining a plurality of breakpoints in the reference genome sequence based on the distribution of the reads in the reference genome sequence, wherein the number of reads has significance at both sides of the breakpoints; determining a detection window in the reference genome based on the plurality of the breakpoints; determining a first parameter based on reads falling in the detection window; and determining whether the copy number variation presents in the genome sample against the detection window based on difference between the first parameter and a preset threshold. Using the computer readable medium, the method of determining whether copy number variation presents in a genome sample according to embodiments of the present disclosure may be effectively implemented, so as to effectively determine whether copy number variation presents in the genome sample, which is suitable for various copy number variations, included but not limited to aneuploidy of chromosome, deletion of chromosome, and addition, micro-deletion and micro-repetition of chromosome fragments.

It should note that, it would be appreciated by those skilled in the art that, the above-described characteristics and advantages of the method of determining whether copy number variation presents in a genome sample is also suitable to the computer readable medium, which are omitted for convenience and brevity.

Reference will be made in detail to examples of the present disclosure. It would be appreciated by those skilled in the art that the following examples are explanatory, and cannot be construed to limit the scope of the present disclosure. If the specific technology or conditions are not specified in the examples, a step will be performed in accordance with the techniques or conditions described in the literature in the art (for example, referring to J. Sambrook, et al. (translated by Huang PT), Molecular Cloning: A Laboratory Manual, 3rd Ed., Science Press) or in accordance with the product instructions. If the manufacturers of reagents or instruments are not specified, the reagents or instruments may be commercially available, for example, from Illumina.

General Method

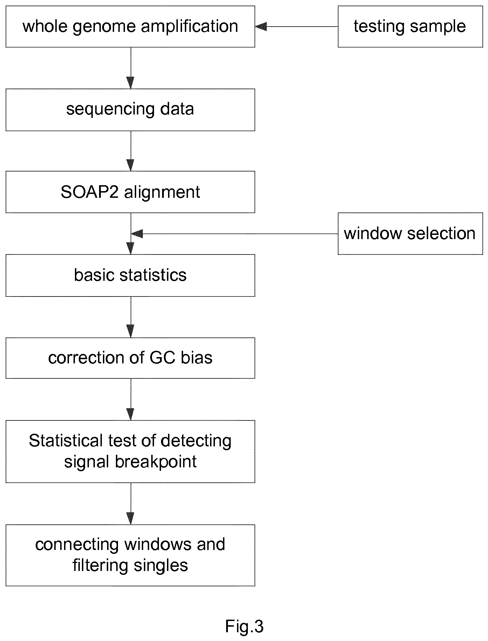

Referring to Fig.3, the method of determining whether copy number variation presents in a genome sample used in examples comprises:

Firstly, a whole genome sample is subjected to amplification, and then the amplified whole genome is sequenced to obtain reads (sequencing data);

Secondly, the obtained reads are aligned to a standard human genome reference sequence in NCBI database by SOAP2, to obtain location information of the reads in the genome. To avoid interference to analysis of copy number variation by repeat sequence, reads which are uniquely aligned to the human genome reference sequence are only selected for subsequent analysis.

Thirdly, a site of which the number of reads falling in two sides respectively having a statistical significance is found, which comprises following steps:

a) calculating the relative number of reads of the testing sample(a plurality of samples may be analyzed at the same time):

a window having a length of w is selected in the human genome reference sequence (w may be any integer greater than 1, for example 10 K to 10 M bp, 50 K to 1 M bp is preferred, 100 K to 300 K bp is more preferred, such as 150 K bp), the number of reads falling in each window $r_{i,j}$ is calculated in al obtained reads, in which subscript i represents No. of the windows, subscript j represents No. of the samples, GC content of each window $GC_{i,j}$ is also calculated, then the relative number of reads is calculated by $R_{i,j} = \log_2\left(\dfrac{r_{i,j}}{\overline{r}_j}\right)$ in , which the average number of reads is $\overline{r}_j = \dfrac{1}{n}\sum_{i=1}^{n} r_{i,j}$ ,

b) data correlation and normalization

in a coordinate system taking GC content as X-coordinate and the relative number of reads R as Y-coordinate, the X-coordinate is divided into a plurality of regions having same units, a mean value $M_s$ of R in every region is calculated, s is No. of GC region;

for every window of the sample, the corrected relative number of reads is calculated by $\tilde{R}_{i,j} = R_{i,j} - M_s$, GC content of window is in the s-th GC region;

for every window of the sample, the normalized relative number of reads $Z_{i,j}$ is calculated by ,

$$Z_{i,j} = \left(R_{i,j} - \tilde{R}_{i,j} - mean_j\right)\Big/ SD_j \ ,$$

in which

$$mean_j = \frac{1}{n}\sum_{i=1}^{n}\left(R_{i,j} - \tilde{R}_{i,j}\right) ,$$

$$SD_j = \sqrt{\frac{1}{n-1}\sum_{i=1}^{n}\left(R_{i,j} - \tilde{R}_{i,j} - mean_j\right)^2} \ ,$$

c) determining and screening breakpoints

determining breakpoints: for each site in the reference genome sequence, n windows (for example 100 windows) are selceted respectively from two sides of the site as two polulations for statistical test, one p value corresponding to each site is obtained by calculating difference between two sides of the site, m sites (such as 3000 sites) having the minimum p value as breakpoint

screening breakpoints: all arranged breakpoints are recorded as $B_c = \{b_1, b_2, ..., b_s\}$, each breakpoint preasents between two successive fragments, in which such two fragments are regions respective from a previous breakpoint to said breakpoint and from said breakpoint to the a next breakpoint, all $Z_{i,j}$ in such two fragments are subjected to statistical test (such as subjected to Run-Test, which is a nonparametric test, evaluating significant difference between two populations using evenly distributed status of mixed elements with two population). The obtained p value ($p_k$) is regarded as "$b_k$ is taken as significance of breakpoint". A candidate breakpoint having the maximum p value $p_k$ is removed, which are repeated until all p value smaller than a final p value $p_{final}$ of such chromosome;

obtaining the final p value: during detection, the above step of determining a plurality breakpoint is performed with a control sample as the testing sample, all arrange candidate breakpoints in whole genome are recorded as $B_c = \{b_1, b_2, ..., b_s\}$, each candidate breakpoint $b_k$ preasents between two successive fragments, all $Z_{i,j}$ in such two fragments are subjected to statistical test, the obtained p value ($p_k$) is regarded as "$b_k$ is taken as significance of breakpoint". A candidate breakpoint having the least significance p value $p_k$ is removed, which are repeated until the number of the candicate breakpoints is zero. A distribution diagram is plotted with the removed candidate breakpoint, a p value having a maximal changing trend is taken as the final p value ($p_{final}$);

determining a detection window and verifying the detection window: after the screened breakpoints have been obtained, the detection window is determined. To further determining the detection window, a mean value of $Z_{i,j}$ in such fragment is calculated, which is recored as $\overline{Z}$. If $\overline{Z}$ exceeds a threshold, then copy number variation is determined presenting in such fragment, in which the threshold is determined as followings:

for each fragment after window combination, a mean value and a standard error of the normalization number of reads $Z_{i,j}$ in such fragment of all control samples are calculated. As $\bar{Z}$ in each frament fits normal distribution, a range of threshold of such fragment when a cumulative probalility is 0.05 is calculated according to the calculated mean value and standard error obtained in above steps, in which the range of threshold is used as the threshold filtering whether copy number variation presents in the fragment.

**Example 1 Copy number variation dection of fetal fragments with an embryo single cell sample, and chromosome aneuploid detection with an embryo single cell sample**

1. whole genome amplification: GenomePlex® Single Cell Whole Genome Amplification Kit from Sigma Aldrich Company was used in whole genome amplification with the two embryo single cell samples in the current example. The embryo single cell sample was trophoblast cell of the fifth day blastocysts, which was isolated from blastaea by a laser capture microdissection method. After the two embryo single cell samples were lysed, the whole genome amplification was performed in accordance with instructions for kit provided by manufacturer.

2. sequencing: in the current example, Hiseq2000 sequencing platform from Illumina Company was used in sequencing the amplified whole genome DNA from the two embryo single cell sample. According to instructions provided by Illumina Company, sequencing-library construction and sequencing on computer were performed, by which generated about 0.36 G data volume of each sample, distinguished by different index sequences. Using alignment software SOAP2, the reads obtained by sequencing were aligned to human genome reference sequencing in NCBI database, Build 36, to locate the obtained reads in the human genome reference sequence.

3. Data analysis

a) calculating the relative number of reads of a testingsample and a control sample (the control sample refered to a sample had normal karyotype)

The human genome reference sequence was divided into a plurality of windows having a length of 150K bp. The number of the reads obtained in step 2) falling in each window $r_{i,j}$ was calculated, in which the subscript i represented No. of the plurality of windows, j represented No. of samples. GC content was also calculated for each window. The relative number of reads was calculated in accordance with the formula given in General Method.

b) data correction and normalization

in a coordinate system taking GC content as X-coordinate and the relative number of reads R as Y-coordinate, the X-coordinate is divided into a plurality of regions having same units, in which the unit is 0.001. A mean value $M_s$ of R in every region was calculated, s was No. of GC region, which were shown in Table.1. The obtained reads were subjected to correction and normalization in accordance to the formula given in General Method.

Table.1 List of $M_s$ in each GC content region during correction

| | Sample S1 | | Sample S2 | | | Sample S1 | | Sample S2 | |
|---|---|---|---|---|---|---|---|---|---|
| **s** | **GC** | $M_s$ | **GC** | $M_s$ | S | GC | $M_s$ | GC | $M_s$ |
| 1 | 0.255~0.256 | 2.45 | 0.255~0.256 | 2.74 | 118 | 0.433~0.434 | -0.23 | 0.452~0.453 | -0.06 |
| 2 | 0.314~0.315 | 0.04 | 0.336~0.337 | -0.26 | 119 | 0.434~0.435 | -0.21 | 0.453~0.454 | -0.15 |
| 3 | 0.317~0.318 | 0.22 | 0.337~0.338 | -0.21 | 120 | 0.435~0.436 | -0.25 | 0.454~0.455 | -0.22 |
| 4 | 0.319~0.32 | 0.01 | 0.338~0.339 | -0.18 | 121 | 0.436~0.437 | -0.25 | 0.455~0.456 | -0.16 |
| 5 | 0.32~0.321 | 0.19 | 0.339~0.34 | 0.16 | 122 | 0.437~0.438 | -0.24 | 0.456~0.457 | -0.19 |
| 6 | 0.321~0.322 | 0.13 | 0.34~0.341 | -0.73 | 123 | 0.438~0.439 | -0.23 | 0.457~0.458 | -0.14 |
| 7 | 0.322~0.323 | 0.11 | 0.341~0.342 | -0.3 | 124 | 0.439~0.44 | -0.29 | 0.458~0.459 | -0.15 |
| 8 | 0.323~0.324 | 0.12 | 0.342~0.343 | -0.28 | 125 | 0.44~0.441 | -0.28 | 0.459~0.46 | -0.21 |
| 9 | 0.324~0.325 | -0.08 | 0.343~0.344 | -0.36 | 126 | 0.441~0.442 | -0.41 | 0.46~0.461 | -0.1 |
| 10 | 0.325~0.326 | 0.02 | 0.344~0.345 | -0.31 | 127 | 0.442~0.443 | -0.28 | 0.461~0.462 | -0.2 |
| 11 | 0.326~0.327 | 0.39 | 0.345~0.346 | -0.19 | 128 | 0.443~0.444 | -0.36 | 0.462~0.463 | -0.19 |
| 12 | 0.327~0.328 | 0.15 | 0.346~0.347 | -0.18 | 129 | 0.444~0.445 | -0.33 | 0.463~0.464 | -0.12 |

(continued)

| s | Sample S1 | | Sample S2 | | S | Sample S1 | | Sample S2 | |
|---|---|---|---|---|---|---|---|---|---|
| | **GC** | $M_s$ | **GC** | $M_s$ | | GC | $M_s$ | GC | $M_s$ |
| 13 | 0.328~0.329 | 0.11 | 0.347~0.348 | -0.25 | 130 | 0.445~0.446 | -0.35 | 0.464~0.465 | -0.3 |
| 14 | 0.329~0.33 | 0.22 | 0.348~0.349 | -0.33 | 131 | 0.446~0.447 | -0.36 | 0.465~0.466 | -0.29 |
| 15 | 0.33~0.331 | 0 | 0.349~0.35 | -0.28 | 132 | 0.447~0.448 | -0.3 | 0.466~0.467 | -0.18 |
| 16 | 0.331~0.332 | -0.04 | 0.35~0.351 | -0.33 | 133 | 0.448~0.449 | -0.47 | 0.467~0.468 | -0.27 |
| 17 | 0.332~0.333 | 0.12 | 0.351~0.352 | -0.14 | 134 | 0.449~0.45 | -0.38 | 0.468~0.469 | -0.24 |
| 18 | 0.333~0.334 | 0.12 | 0.352~0.353 | -0.24 | 135 | 0.45~0.451 | -0.43 | 0.469~0.47 | -0.28 |
| 19 | 0.334~0.335 | 0.06 | 0.353~0.354 | -0.23 | 136 | 0.451~0.452 | -0.4 | 0.47~0.471 | -0.25 |
| 20 | 0.335~0.336 | 0.14 | 0.354~0.355 | -0.15 | 137 | 0.452~0.453 | -0.34 | 0.471~0.472 | -0.24 |
| 21 | 0.336~0.337 | 0.1 | 0.355~0.356 | -0.21 | 138 | 0.453~0.454 | -0.5 | 0.472~0.473 | -0.44 |
| 22 | 0.337~0.338 | 0.08 | 0.356~0.357 | -0.19 | 139 | 0.454~0.455 | -0.45 | 0.473~0.474 | -0.37 |
| 23 | 0.338~0.339 | 0.08 | 0.357~0.358 | -0.18 | 140 | 0.455~0.456 | -0.5 | 0.474~0.475 | -0.36 |
| 24 | 0.339~0.34 | 0.1 | 0.358~0.359 | -0.14 | 141 | 0.456~0.457 | -0.47 | 0.475~0.476 | -0.31 |
| 25 | 0.34~0.341 | 0.15 | 0.359~0.36 | -0.09 | 142 | 0.457~0.458 | -0.49 | 0.476~0.477 | -0.41 |
| 26 | 0.341~0.342 | 0.12 | 0.36~0.361 | -0.15 | 143 | 0.458~0.459 | -0.47 | 0.477~0.478 | -0.41 |
| 27 | 0.342~0.343 | 0.11 | 0.361~0.362 | -0.13 | 144 | 0.459~0.46 | -0.52 | 0.478~0.479 | -0.41 |
| 28 | 0.343~0.344 | 0.06 | 0.362~0.363 | -0.13 | 145 | 0.46~0.461 | -0.58 | 0.479~0.48 | -0.36 |
| 29 | 0.344~0.345 | 0.17 | 0.363~0.364 | -0.09 | 146 | 0.461~0.462 | -0.61 | 0.48~0.481 | -0.44 |
| 30 | 0.345~0.346 | 0.09 | 0.364~0.365 | -0.13 | 147 | 0.462~0.463 | -0.64 | 0.481~0.482 | -0.37 |
| 31 | 0.346~0.347 | 0.14 | 0.365~0.366 | -0.08 | 148 | 0.463~0.464 | -0.55 | 0.482~0.483 | -0.38 |
| 32 | 0.347~0.348 | 0.08 | 0.366~0.367 | -0.06 | 149 | 0.464~0.465 | -0.57 | 0.483~0.484 | -0.46 |
| 33 | 0.348~0.349 | 0.11 | 0.367~0.368 | -0.06 | 150 | 0.465~0.466 | -0.68 | 0.484~0.485 | -0.52 |
| 34 | 0.349~0.35 | 0.13 | 0.368~0.369 | -0.08 | 151 | 0.466~0.467 | -0.57 | 0.485~0.486 | -0.57 |
| 35 | 0.35~0.351 | 0.08 | 0.369~0.37 | -0.06 | 152 | 0.467~0.468 | -0.78 | 0.486~0.487 | -0.47 |
| 36 | 0.351~0.352 | 0.14 | 0.37~0.371 | -0.09 | 153 | 0.468~0.469 | -0.75 | 0.487~0.488 | -0.55 |
| 37 | 0.352~0.353 | 0.13 | 0.371~0.372 | -0.03 | 154 | 0.469~0.47 | -0.64 | 0.488~0.489 | -0.45 |
| 38 | 0.353~0.354 | 0.12 | 0.372~0.373 | -0.01 | 155 | 0.47~0.471 | -0.74 | 0.489~0.49 | -0.74 |
| 39 | 0.354~0.355 | 0.13 | 0.373~0.374 | -0.03 | 156 | 0.471~0.472 | -0.57 | 0.49~0.491 | -0.52 |
| 40 | 0.355~0.356 | 0.12 | 0.374~0.375 | -0.06 | 157 | 0.472~0.473 | -0.69 | 0.491~0.492 | -0.59 |
| 41 | 0.356~0.357 | 0.15 | 0.375~0.376 | -0.04 | 158 | 0.473~0.474 | -0.73 | 0.492~0.493 | -0.57 |
| 42 | 0.357~0.358 | 0.14 | 0.376~0.377 | -0.04 | 159 | 0.474~0.475 | -0.74 | 0.493~0.494 | -0.59 |
| 43 | 0.358~0.359 | 0.16 | 0.377~0.378 | -0.01 | 160 | 0.475~0.476 | -0.84 | 0.494~0.495 | -0.54 |
| 44 | 0.359~0.36 | 0.14 | 0.378~0.379 | -0.01 | 161 | 0.476~0.477 | -0.79 | 0.495~0.496 | -0.63 |
| 45 | 0.36~0.361 | 0.14 | 0.379~0.38 | 0 | 162 | 0.477~0.478 | -0.81 | 0.496~0.497 | -0.69 |
| 46 | 0.361~0.362 | 0.14 | 0.38~0.381 | -0.01 | 163 | 0.478~0.479 | -0.78 | 0.497~0.498 | -0.63 |
| 47 | 0.362~0.363 | 0.15 | 0.381~0.382 | 0.03 | 164 | 0.479~0.48 | -0.71 | 0.498~0.499 | -0.7 |
| 48 | 0.363~0.364 | 0.09 | 0.382~0.383 | 0 | 165 | 0.48~0.481 | -0.94 | 0.499~0.5 | -0.69 |
| 49 | 0.364~0.365 | 0.1 | 0.383~0.384 | 0.01 | 166 | 0.481~0.482 | -0.8 | 0.5~0.501 | -0.64 |

(continued)

| | Sample S1 | | Sample S2 | | | Sample S1 | | Sample S2 | |
|---|---|---|---|---|---|---|---|---|---|
| **s** | **GC** | $M_s$ | **GC** | $M_s$ | S | GC | $M_s$ | GC | $M_s$ |
| 50 | 0.365~0.366 | 0.14 | 0.384~0.385 | 0 | 167 | 0.482~0.483 | -0.74 | 0.501~0.502 | -0.75 |
| 51 | 0.366~0.367 | 0.12 | 0.385~0.386 | 0.04 | 168 | 0.483~0.484 | -0.78 | 0.502~0.503 | -0.71 |
| 52 | 0.367~0.368 | 0.11 | 0.386~0.387 | 0.03 | 169 | 0.484~0.485 | -0.95 | 0.503~0.504 | -0.85 |
| 53 | 0.368~0.369 | 0.12 | 0.387~0.388 | 0.03 | 170 | 0.485~0.486 | -0.81 | 0.504~0.505 | -0.67 |
| 54 | 0.369~0.37 | 0.15 | 0.388~0.389 | 0.04 | 171 | 0.486~0.487 | -0.96 | 0.505~0.506 | -0.97 |
| 55 | 0.37~0.371 | 0.15 | 0.389~0.39 | 0.03 | 172 | 0.487~0.488 | -1 | 0.506~0.507 | -0.81 |
| 56 | 0.371~0.372 | 0.14 | 0.39~0.391 | 0.05 | 173 | 0.488~0.489 | -0.91 | 0.507~0.508 | -0.72 |
| 57 | 0.372~0.373 | 0.09 | 0.391~0.392 | 0.02 | 174 | 0.489~0.49 | -0.86 | 0.508~0.509 | -0.75 |
| 58 | 0.373~0.374 | 0.11 | 0.392~0.393 | 0.03 | 175 | 0.49~0.491 | -0.85 | 0.509~0.51 | -0.6 |
| 59 | 0.374~0.375 | 0.13 | 0.393~0.394 | 0.05 | 176 | 0.491~0.492 | -1.01 | 0.51~0.511 | -0.78 |
| 60 | 0.375~0.376 | 0.11 | 0.394~0.395 | 0.07 | 177 | 0.492~0.493 | -1.11 | 0.511~0.512 | -0.76 |
| 61 | 0.376~0.377 | 0.12 | 0.395~0.396 | 0.05 | 178 | 0.493~0.494 | -0.94 | 0.512~0.513 | -0.75 |
| 62 | 0.377~0.378 | 0.13 | 0.396~0.397 | 0.07 | 179 | 0.494~0.495 | -1.01 | 0.513~0.514 | -0.82 |
| 63 | 0.378~0.379 | 0.08 | 0.397~0.398 | 0.06 | 180 | 0.495~0.496 | -0.95 | 0.514~0.515 | -0.75 |
| 64 | 0.379~0.38 | 0.13 | 0.398~0.399 | 0.03 | 181 | 0.496~0.497 | -0.99 | 0.515~0.516 | -1.15 |
| 65 | 0.38~0.381 | 0.08 | 0.399~0.4 | 0.08 | 182 | 0.497~0.498 | -1.09 | 0.516~0.517 | -0.68 |
| 66 | 0.381~0.382 | 0.06 | 0.4~0.401 | 0.08 | 183 | 0.498~0.499 | -1.17 | 0.517~0.518 | -0.73 |
| 67 | 0.382~0.383 | 0.12 | 0.401~0.402 | 0.1 | 184 | 0.499~0.5 | -0.96 | 0.518~0.519 | -1.07 |
| 68 | 0.383~0.384 | 0.1 | 0.402~0.403 | 0.09 | 185 | 0.5~0.501 | -1.02 | 0.519~0.52 | -1 |
| 69 | 0.384~0.385 | 0.11 | 0.403~0.404 | 0.08 | 186 | 0.501~0.502 | -1.06 | 0.52~0.521 | -0.93 |
| 70 | 0.385~0.386 | 0.08 | 0.404~0.405 | 0.09 | 187 | 0.502~0.503 | -1.13 | 0.521~0.522 | -0.99 |
| 71 | 0.386~0.387 | 0.07 | 0.405~0.406 | 0.09 | 188 | 0.503~0.504 | -1.48 | 0.522~0.523 | -1 |
| 72 | 0.387~0.388 | 0.07 | 0.406~0.407 | 0.1 | 189 | 0.504~0.505 | -1.16 | 0.523~0.524 | -1.01 |
| 73 | 0.388~0.389 | 0.07 | 0.407~0.408 | 0.06 | 190 | 0.505~0.506 | -0.8 | 0.524~0.525 | -1.17 |
| 74 | 0.389~0.39 | 0.07 | 0.408~0.409 | 0.07 | 191 | 0.506~0.507 | -1.22 | 0.525~0.526 | -1.13 |
| 75 | 0.39~0.391 | 0.1 | 0.409~0.41 | 0.08 | 192 | 0.507~0.508 | -1.06 | 0.526~0.527 | -1.14 |
| 76 | 0.391~0.392 | 0.06 | 0.41~0.411 | 0.06 | 193 | 0.508~0.509 | -1.31 | 0.527~0.528 | -0.73 |
| 77 | 0.392~0.393 | 0.06 | 0.411~0.412 | 0.05 | 194 | 0.509~0.51 | -1.27 | 0.528~0.529 | -1.01 |
| 78 | 0.393~0.394 | 0.06 | 0.412~0.413 | 0.09 | 195 | 0.51~0.511 | -1.05 | 0.529~0.53 | -1.15 |
| 79 | 0.394~0.395 | 0.05 | 0.413~0.414 | 0.06 | 196 | 0.511~0.512 | -1.37 | 0.53~0.531 | -1.03 |
| 80 | 0.395~0.396 | 0.04 | 0.414~0.415 | 0.08 | 197 | 0.512~0.513 | -1.39 | 0.531~0.532 | -1.06 |
| 81 | 0.396~0.397 | 0.06 | 0.415~0.416 | 0.05 | 198 | 0.513~0.514 | -1.43 | 0.532~0.533 | -1.05 |
| 82 | 0.397~0.398 | 0.03 | 0.416~0.417 | 0.04 | 199 | 0.514~0.515 | -1.45 | 0.533~0.534 | -1.42 |
| 83 | 0.398~0.399 | 0.02 | 0.417~0.418 | 0.09 | 200 | 0.515~0.516 | -1.3 | 0.534~0.535 | -0.89 |
| 84 | 0.399~0.4 | 0.09 | 0.418~0.419 | 0.06 | 201 | 0.516~0.517 | -1.38 | 0.535~0.536 | -1.8 |
| 85 | 0.4~0.401 | 0.02 | 0.419~0.42 | -0.01 | 202 | 0.517~0.518 | -0.94 | 0.536~0.537 | -0.81 |
| 86 | 0.401~0.402 | 0.01 | 0.42~0.421 | 0.09 | 203 | 0.518~0.519 | -1.48 | 0.537~0.538 | -0.89 |

(continued)

| | Sample S1 | | Sample S2 | | | Sample S1 | | Sample S2 | |
|---|---|---|---|---|---|---|---|---|---|
| s | GC | $M_s$ | GC | $M_s$ | S | GC | $M_s$ | GC | $M_s$ |
| 87 | 0.402~0.403 | 0.03 | 0.421~0.422 | 0.08 | 204 | 0.519~0.52 | -1.48 | 0.538~0.539 | -0.91 |
| 88 | 0.403~0.404 | 0 | 0.422~0.423 | 0.06 | 205 | 0.52~0.521 | -0.91 | 0.539~0.54 | -0.96 |
| 89 | 0.404~0.405 | 0.03 | 0.423~0.424 | 0.08 | 206 | 0.521~0.522 | -0.89 | 0.54~0.541 | -1.98 |
| 90 | 0.405~0.406 | 0.02 | 0.424~0.425 | 0.03 | 207 | 0.522~0.523 | -1.9 | 0.541~0.542 | -0.29 |
| 91 | 0.406~0.407 | 0.03 | 0.425~0.426 | 0.06 | 208 | 0.523~0.524 | -1.46 | 0.542~0.543 | -1.28 |
| 92 | 0.407~0.408 | 0.02 | 0.426~0.427 | 0.05 | 209 | 0.524~0.525 | -2.02 | 0.543~0.544 | -1.84 |
| 93 | 0.408~0.409 | -0.01 | 0.427~0.428 | 0.06 | 210 | 0.525~0.526 | -1.39 | 0.544~0.545 | -1.41 |
| 94 | 0.409~0.41 | -0.06 | 0.428~0.429 | 0.03 | 211 | 0.526~0.527 | -1.72 | 0.545~0.546 | -0.54 |
| 95 | 0.41~0.411 | -0.06 | 0.429~0.43 | 0.04 | 212 | 0.528~0.529 | -1.08 | 0.547~0.548 | -1.31 |
| 96 | 0.411~0.412 | -0.04 | 0.43~0.431 | 0.05 | 213 | 0.529~0.53 | -1.42 | 0.548~0.549 | -1.11 |
| 97 | 0.412~0.413 | -0.04 | 0.431~0.432 | 0.01 | 214 | 0.53~0.531 | -1.71 | 0.549~0.55 | -1.38 |
| 98 | 0.413~0.414 | -0.02 | 0.432~0.433 | 0.04 | 215 | 0.531~0.532 | -2.27 | 0.55~0.551 | -1.5 |
| 99 | 0.414~0.415 | -0.05 | 0.433~0.434 | 0 | 216 | 0.532~0.533 | -1.78 | 0.551~0.552 | -1.22 |
| 100 | 0.415~0.416 | -0.07 | 0.434~0.435 | -0.02 | 217 | 0.533~0.534 | -1.55 | 0.552~0.553 | -0.8 |
| 101 | 0.416~0.417 | -0.08 | 0.435~0.436 | 0.01 | 218 | 0.535~0.536 | -1.25 | 0.553~0.554 | -1.32 |
| 102 | 0.417~0.418 | -0.11 | 0.436~0.437 | 0.04 | 219 | 0.536~0.537 | -1.09 | 0.554~0.555 | -1.79 |
| 103 | 0.418~0.419 | -0.07 | 0.437~0.438 | 0.01 | 220 | 0.537~0.538 | -2.02 | 0.556~0.557 | -1.3 |
| 104 | 0.419~0.42 | -0.09 | 0.438~0.439 | -0.01 | 221 | 0.54~0.541 | -2.16 | 0.557~0.558 | -1.48 |
| 105 | 0.42~0.421 | -0.13 | 0.439~0.44 | -0.01 | 222 | 0.541~0.542 | -1.64 | 0.558~0.559 | -1.7 |
| 106 | 0.421~0.422 | -0.1 | 0.44~0.441 | -0.01 | 223 | 0.544~0.545 | -2.3 | 0.559~0.56 | -1.55 |
| 107 | 0.422~0.423 | -0.12 | 0.441~0.442 | -0.01 | 224 | 0.546~0.547 | -2.51 | 0.561~0.562 | -1.62 |
| 108 | 0.423~0.424 | -0.11 | 0.442~0.443 | -0.06 | 225 | 0.548~0.549 | -2.7 | 0.563~0.564 | -1.68 |
| 109 | 0.424~0.425 | -0.17 | 0.443~0.444 | -0.04 | 226 | 0.549~0.55 | -1.77 | 0.564~0.565 | -1.47 |
| 110 | 0.425~0.426 | -0.14 | 0.444~0.445 | -0.07 | 227 | 0.55~0.551 | -1.08 | 0.569~0.57 | -1.42 |
| 111 | 0.426~0.427 | -0.14 | 0.445~0.446 | -0.11 | 228 | 0.551~0.552 | -2.13 | 0.58~0.581 | -1.74 |
| 112 | 0.427~0.428 | -0.15 | 0.446~0.447 | -0.13 | 229 | 0.553~0.554 | -2.19 | 0.583~0.584 | -2.43 |
| 113 | 0.428~0.429 | -0.19 | 0.447~0.448 | -0.08 | 230 | 0.555~0.556 | -2.04 | 0.6~0.601 | -1.79 |
| 114 | 0.429~0.43 | -0.18 | 0.448~0.449 | -0.11 | 231 | 0.556~0.557 | -1.93 | | |
| 115 | 0.43~0.431 | -0.18 | 0.449~0.45 | -0.07 | 232 | 0.562~0.563 | -2.51 | | |
| 116 | 0.431~0.432 | -0.21 | 0.45~0.451 | -0.16 | 233 | 0.572~0.573 | -1.85 | | |
| 117 | 0.432~0.433 | -0.26 | 0.451~0.452 | -0.08 | 234 | 0.574~0.575 | -2.74 | | |

c) Window combination

determining breakpoint, for each site in the reference genome sequence, 100 windows located at either side of the site were selected respectively from two sides of the site as two populations for Run-Test, one p value corresponding to each site was obtained by calculating difference between two sides of the site, 3000 sites having the minimum p value as breakpoint.

screening breakpoint: all arranged breakpoints were recorded as $B_c = \{b_1, b_2, ..., b_s\}$, each breakpoint presented between two successive fragments, in which such two fragments were regions respective from a previous breakpoint to said breakpoint and from said breakpoint to the a next breakpoint, all $Z_{i,j}$ in such two fragments were subjected to Run-Test. The obtained p value ($p_k$) was regarded as "$b_k$ was taken as significance of breakpoint". A candidate breakpoint having the maximum p *value $p_k$* was removed, which were repeated until all p value smaller than the final p value $p_{final}$ of such chromosome being as $1.1 \times 10^{-50}$.

d) after the breakpoints were screened out, a region between two successive breakpoints was determined as a detection window, for window combination. To further filter fragments obtained by window combination, a mean value of $Z_{i,j}$ in such fragment was calculated, which was recorded as $\overline{Z}$. If $\overline{Z}$ exceeded a threshold, then copy number variation was determined presenting in such fragment. -1.645 was used as the first threshold, and 1.645 was used as the second threshold.

4. Result

Table.2 showed a detection result list of copy number variation after whole genome amplifying the embryo single cell sample in current example.

Table.2 Detection result list of copy number variation after whole genome amplifying the embryo single cell sample in current example

| No. | chromosome | starting point of CNV | terminating point of CNV | size of CNV | type of CNV | Involved region |
|---|---|---|---|---|---|---|
| S1 | 5 | 63,429 | 23,496,649 | 23.4M | deletion | 4q34.3→q35.2 |
| | 12 | 16,037 | 18,926,068 | 18.9M | repeat | 7p21.1→p22.3 |
| S2 | 21 | 1 | 46,944,323 | 46.9M | repeat | 21p13→q22.3 |

It could be seen from Table.2 that using the method of detemining whether copy number variation presents in a genome sample according to embodiments of the present disclosure, various types of copy number variation could be effectively determined.

Example 2

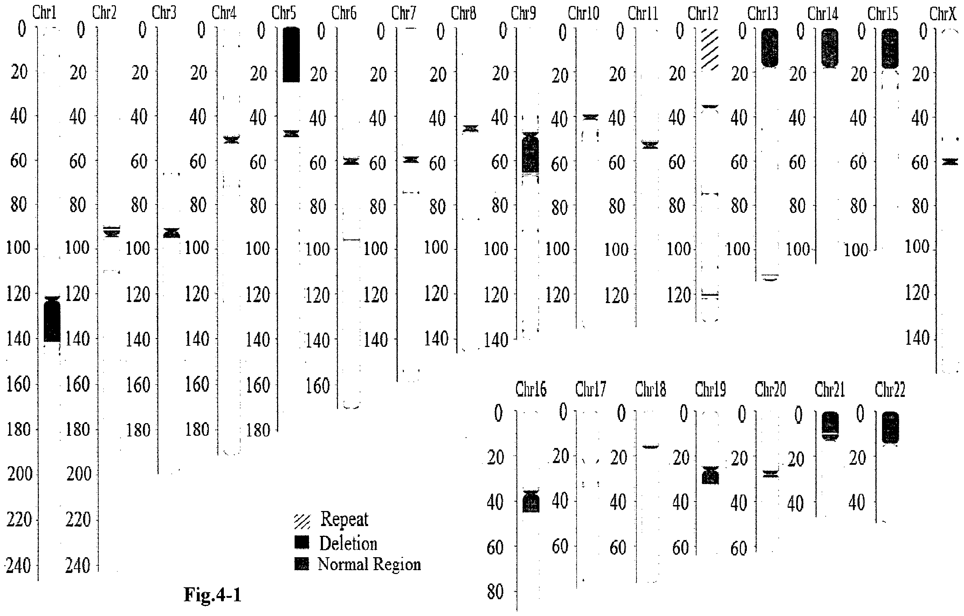

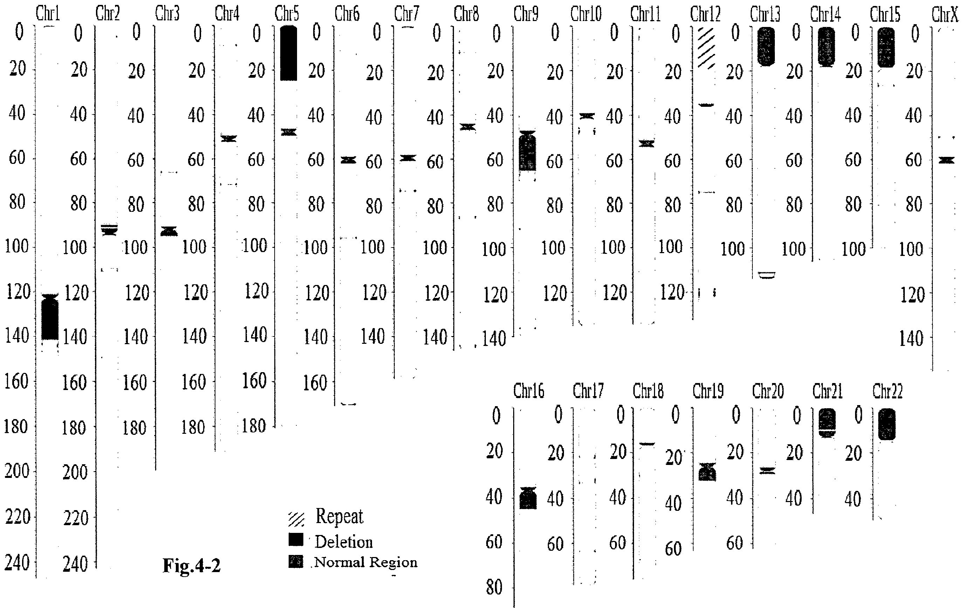

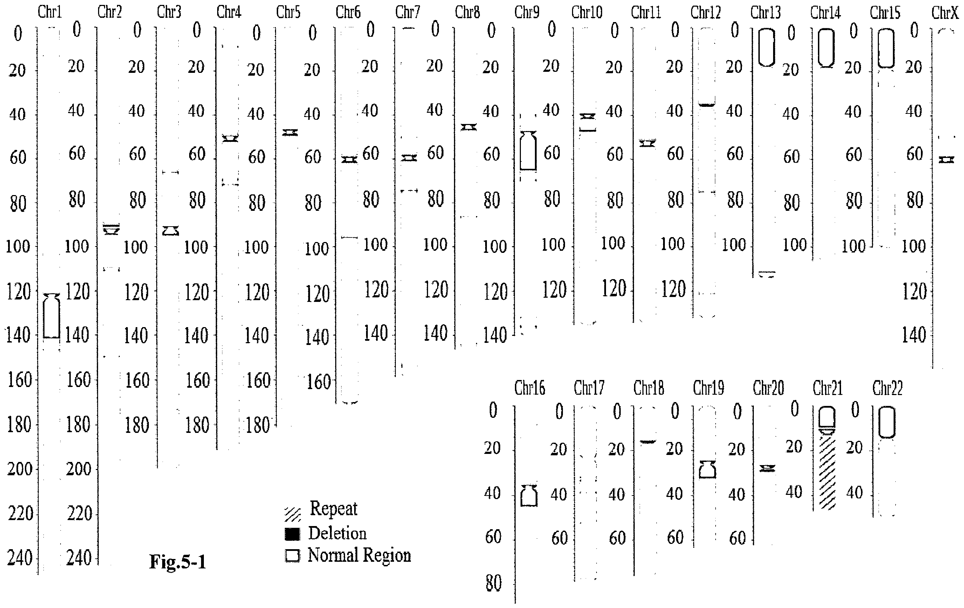

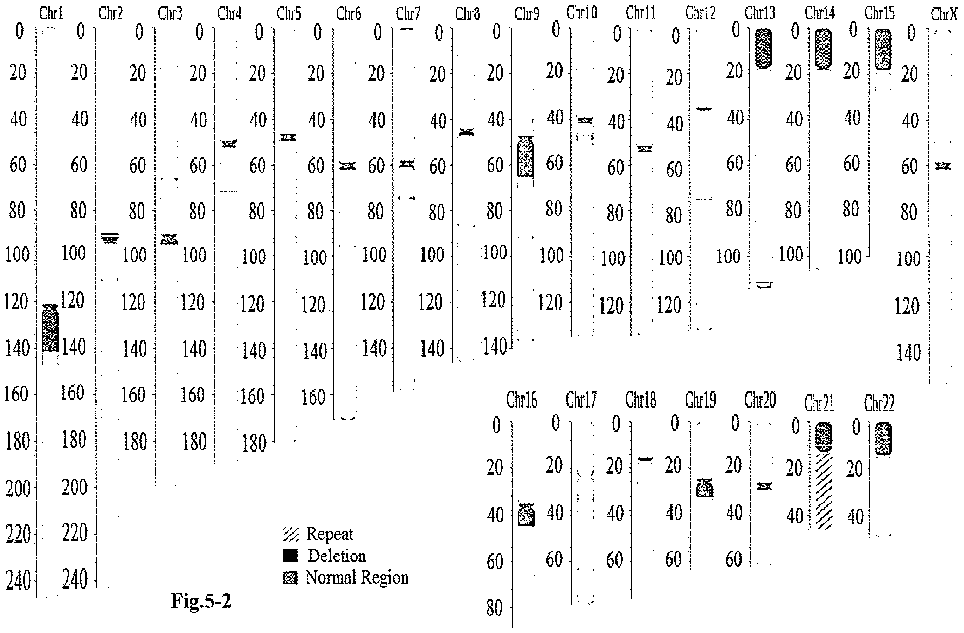

Using the embryo single cell sample same as that in Example 1, all steps were rereated as Example 1 except the genome DNA was directly subjected to sequencing (without firstly subjected to whole genome amplification). Comparision result between Example 1 and Example 2 was shown in Table 3, Fig.4 and Fig.5.

Table 3. Comparison result of detecting copy number variation of reads obtained by subjected each genome sample to whole genome amplification and not to whole genome amplification

| No. | chromosome | sequencing result of embryo single cell genome DNA (without subjected to WGA) | sequencing result of embryo single cell genome DNA by the method of the present disclosure | determining result |
|---|---|---|---|---|
| S1 | 5 | deletion: 10,002-23,312,155 | Deletion: 63,429-23,496,649 | consistent |
| | 12 | repeat: 145,741-14,780,155 | repeat: 16,037-18,926,068 | consistent |
| S2 | 21 | 21 trisomy | 21 trisomy | consistent |

It could be seen from data in Table.3 and images of chromosome karyotype in Fig.4 and Fig.5 that the detection results of reads copy number variation between the genome DNA sample which was subjected to whole genome amplificaiton and the genome DNA sample which was not subjected to whole genome amplificaiton were consistent. For difference of staring and terminating points of "deletion" or "repeat" in Table.3, as the boundary of copy number variation was hard to be accurately determined, in general for the primary window having a length of about 150K, two boundaries having difference within a range of 100 to 300 Kb could be determined as being fully consistent, two boundaries having difference within a range of 300Kb to 1 Mb could be determined as being quite consistent. Since the difference between bondaries of copy number variation determined by the two methods in Table 3. was within the range of 100 to 300 Kb or within the range of 300Kb to 1 Mb, it could determine that the boundaries of copy number variation determined by the two methods were consistent.

Industrial applicability

The method, system and computer readable medium of determining whether copy number variation presents in a genome sample of the present disclosure may be effectively used to determine whether copy number variation presents in a genome sample.

Reference throughout this specification to "an embodiment," "some embodiments," "one embodiment", "another example," "an example," "a specific examples" or "some examples," means that a particular feature, structure, material, or characteristic described in connection with the embodiment or example is included in at least one embodiment or example of the present disclosure. Thus, the appearances of the phrases such as "in some embodiments," "in one embodiment", "in an embodiment", "in another example, "in an example," "in a specific examples," or "in some examples," in various places throughout this specification are not necessarily referring to the same embodiment or example of the present disclosure. Furthermore, the particular features, structures, materials, or characteristics may be combined in any suitable manner in one or more embodiments or examples.

## Claims

1. A method of determining whether copy number variation exists in a genome sample, comprising:

sequencing the genome sample, to obtain a sequencing result consisting of a plurality of reads, wherein the genome sample is obtained from a single cell;
aligning the sequencing result to a reference genome sequence, to determine a distribution of the reads in the reference genome sequence;
determining a plurality of breakpoints in the reference genome sequence based on the distribution of the reads in the reference genome sequence, wherein the number of reads has significance at both sides of the breakpoints;
determining a detection window in the reference genome based on the plurality of the breakpoints;
determining a first parameter based on reads falling in the detection window; and
determining whether the copy number variation exists in the genome sample against the detection window based on a difference between the first parameter and a preset threshold;
wherein the step of determining a plurality of breakpoints in the reference genome sequence comprises the steps of
dividing the reference genome sequence into a plurality of primary windows having a predetermined length, and determining reads falling in each of the plurality of the primary windows;
for at least one site in the reference genome sequence, determining the number of reads falling in the same number of the plurality of primary windows at both sides of the site; and
determining a p value of the site, wherein the p value represents that the number of reads falling in either side of the site has significance; wherein the site is the breakpoint, if the p value of the site is smaller than a final p value; wherein the step of determining the p value of the site further comprises:

for the site, selecting the plurality of primary windows having the same number at either side of the site respectively, and calculating the relative number of reads falling in each of the plurality of primary windows $R_i$, wherein i represents the number of the plurality of primary windows,
subjecting all of the relative numbers of reads falling in the plurality of primary windows $R_i$ to a Run-Test, to thereby determine the p value of the site,
wherein the relative number of reads is determined by following formula:

$$R_i = \log_2\left(\frac{r_i}{\bar{r}}\right)$$

wherein $r_i$ represents the number of reads falling in the i-th primary window,

$$\overline{r} = \frac{1}{n} \sum_{i=1}^{n} r_i,$$

n represents the total number of the plurality of primary windows.

2. The method of claim 1, wherein the method further comprises:

lysing the single cell, to release whole genome of the single cell; and
amplifying the whole genome to obtain the genome sample.

3. The method of claim 1 or 2, wherein the copy number variation is at least one selected from the group consisting of aneuploidy of chromosome, deletion of chromosome, addition of a chromosome fragment, micro-deletion of a chromosome fragment and micro-repetition of a chromosome fragment.

4. The method of any one of the previous claims, wherein the reads falling into each of the primary windows are preferably uniquely-aligned reads,
preferably, 100 of the primary windows are selected from either side of the site,
preferably, the primary windows have a length of 100 Kbp to 200 Kbp, more preferably 150 Kbp,
preferably, the final p value is $1.1 \times 10^{-50}$ or less.

5. The method of claim 1, wherein subjecting all of the relative numbers of the reads falling in each of the plurality of primary windows to Run-Test further comprises:

subjecting the relative number of reads falling in each of the plurality of primary windows $R_i$ to a correction of GC content, to obtain a corrected relative number of reads $\tilde{R}_i$;
determining a normalized number of reads falling in each of the plurality of primary windows $Z_i$ based on the corrected relative number of reads; and
subjecting all of the normalized numbers of reads falling in each of the plurality of primary windows $Z_i$ to a Run-Test.

6. The method of claim 5, wherein the corrected relative number of reads $\tilde{R}_i$ is obtained by the following steps:

calculating a GC content of each of the plurality of primary windows;
dividing the GC content into a plurality of regions in a unit of 0.001, and calculating a mean value $M_s$ among all of the relative numbers of reads falling in each of the plurality of regions, wherein s represents the number of the plurality of regions;
determining the corrected relative number of read $\tilde{R}_i$ based on the following formula:

$$\tilde{R}_i = R_i - M_s;$$

and
determining the normalized number of reads $Z_i$ based on the following formula: wherein

$$Z_i = \left( R_i - \tilde{R}_i - mean \right)/SD,$$

wherein

$$mean = \frac{1}{n} \sum_{i=1}^{n} \left( R_i - \tilde{R}_i \right),$$

and

$$SD = \sqrt{\frac{1}{n-1}\sum_{i=1}^{n}\left(R_i - \tilde{R}_i - mean\right)^2}$$ .

7. The method of any one of the previous claims, wherein the step of determining a detection window in the reference genome based on the plurality of the breakpoints further comprises:

1) determining a plurality of candidate breakpoints, wherein other breakpoints exist both before and after the candidate breakpoints;
2) determining a p value of each candidate breakpoint, and removing a candidate breakpoint having the final p value;
3) performing the step 2) with the rest of the candidate breakpoints until all p values of the rest of the candidate breakpoints are all smaller than the final p value, wherein the rest of the candidate breakpoints are taken as screened candidate breakpoints; and
4) determining a region between two successive screened candidate breakpoints as the detection window,

wherein the p value of the candidate breakpoint is obtained by the following steps:

selecting a region between the candidate breakpoint and a previous candidate breakpoint as a first candidate region, and selecting a region between the candidate breakpoint and a next candidate breakpoint as a second candidate region;
subjecting the normalized number of reads falling in the primary windows Zi which are included both in the first candidate region and the second candidate region to a Run-Test, to thereby determine the p value of the candidate breakpoints.

8. The method of claim 7, wherein the step of determining a first parameter based on the reads falling in the detection window further comprises:

determining a mean value among all of the normalized numbers of reads falling in each of the plurality of primary windows $\overline{Z}$ which are included in the detection windows, wherein the mean value of the normalized numbers of reads $\overline{Z}$ is taken as the first parameter.

9. The method of claim 1, wherein the preset threshold comprises:

a first threshold being -1.645 and a second threshold being 1.645.

10. The method of any one of the previous claims, wherein the reference genome sequence is at least one selected from the group consisting of the sequence of human chromosome 21, human chromosome 18, human chromosome 13, human chromosome X, and human chromosome Y.

11. A system for determining whether copy number variation exists in a genome sample, comprising:

a sequencing apparatus, configured to sequence the genome sample, for obtaining a sequencing result consisting of a plurality of reads, wherein the genome sample is obtained from a single cell;
an analysis apparatus, connected to the sequencing apparatus, and configured to determine whether copy number variation exists in the genome sample based on the sequencing result, wherein the analysis apparatus further comprises:

an aligning unit, configured to align the sequencing result to a reference genome sequence, for determining a distribution of the reads in the reference genome sequence;
a breakpoint determining unit, connected to the aligning unit, and configured to determine a plurality of breakpoints in the reference genome sequence based on the distribution of the reads in the reference genome sequence, wherein the number of reads has significance between two sides of the breakpoints;

a detection window determining unit, connected to the breakpoint determining unit, and configured to determine a detection window in the reference genome based on the plurality of the breakpoints;
a parameter determining unit, connected to the detection window determining unit, and configured to determine a first parameter based on reads falling in the detection window; and
a determining unit, connected to the parameter determining unit, and configured to determine whether the copy number variation exists in the genome sample against the detection window based on a difference between the first parameter and a preset threshold,
wherein the breakpoint determining unit is configured
to divide the reference genome sequence into a plurality of primary windows having a predetermined length, and to determine reads falling in each of the plurality of the primary windows;
to determine for at least one site in the reference genome sequence the number of reads falling in the same number of the plurality of primary windows at both sides of the site; and
to determine a p value of the site, wherein the p value represents that the number of reads falling in either side of the site has significance; wherein the site is the breakpoint, if the p value of the site is smaller than a final p value; wherein the step of determining the p value of the site further comprises:

for the site, selecting the plurality of primary windows having the same number at either side of the site respectively, and calculating the relative number of reads falling in each of the plurality of primary windows $R_i$, wherein i represents the number of the plurality of primary windows,
subjecting all of the relative numbers of reads falling in the plurality of primary windows $R_i$ to a Run-Test, to thereby determine the p value of the site,
wherein the relative number of reads is determined by following formula:

$$R_i = \log_2\left(\frac{r_i}{\overline{r}}\right)$$

wherein $r_i$ represents the number of reads falling in the i-th primary window,

$$\overline{r} = \frac{1}{n}\sum_{i=1}^{n} r_i,$$

n represents the total number of the plurality of primary windows.

12. The system of claim 11, further comprising a genome extracting apparatus, configured to extract the genome sample from a biological sample.

13. The system of claim 11 or 12, wherein the sequencing apparatus further comprises:

a genome amplifying unit, configured to amplify the genome sample;
a sequencing-library constructing unit, connected to the genome amplifying unit, and configured to construct a sequencing-library with amplified genome sample; and
a sequencing unit, connected to the sequencing-library constructing unit, and configured to sequence the sequencing-library.

14. A computer readable medium, comprising an order, configured to perform by a processer to determine whether copy number variation exists in a genome sample through the following steps:

aligning a sequencing result to a reference genome sequence, to determine a distribution of reads in a reference genome sequence;
determining a plurality of breakpoints in the reference genome sequence based on the distribution of the reads in the reference genome sequence, wherein the number of reads has significance at both sides of the breakpoints;
determining a detection window in the reference genome based on the plurality of the breakpoints;
determining a first parameter based on reads falling in the detection window; and
determining whether the copy number variation exists in the genome sample against the detection window based on a difference between the first parameter and a preset threshold,
wherein optionally the step of determining a plurality of breakpoints in the reference genome sequence further comprises:

dividing the reference genome sequence into a plurality of primary windows having a predetermined length, and determining reads falling in each of the plurality of the primary windows;

for at least one site in the reference genome sequence, determining the number of reads falling in the same number of the plurality of primary windows at both sides of the site;

determining a p value of the site, wherein the p value represents that the number of reads falling in either side of the site has significance; and

wherein if the p value of the site is smaller than a final p value, the site is the breakpoints;

wherein the step of determining the p value of the site further comprises:

for the site, selecting the plurality of primary windows having the same number at either side of the site respectively, and calculating the relative number of reads falling in each of the plurality of primary windows $R_i$, wherein i represents the number of the plurality of primary windows,

subjecting all of the relative numbers of reads falling in the plurality of primary windows $R_i$ to a Run-Test, to thereby determine the p value of the site,

wherein the relative number of reads is determined by following formula:

$$R_i = \log_2\left(\frac{r_i}{\bar{r}}\right)$$

wherein $r_i$ represents the number of reads falling in the i-th primary window,

$$\bar{r} = \frac{1}{n}\sum_{i=1}^{n} r_i \, ,$$

n represents the total number of the plurality of primary windows.

**Patentansprüche**

1. Verfahren zur Bestimmung der Existenz einer Kopiezahlvariante in einer Genomprobe, umfassend:

Sequenzieren der Genomprobe, um ein Sequenzierungsergebnis, das aus einer Vielzahl von Reads besteht, zu erhalten, wobei die Genomprobe aus einer einzelnen Zelle gewonnen wird;

Alignment des Sequenzierungsergebnisses mit einer Referenzgenomsequenz, um eine Verteilung der Reads in der Referenzgenomsequenz zu bestimmen;

Bestimmen einer Vielzahl von Bruchpunkten in der Referenzgenomsequenz basierend auf der Verteilung der Reads in der Referenzgenomsequenz, wobei die Anzahl der Reads Signifikanz auf beiden Seiten der Bruchpunkte hat;

Bestimmen eines Nachweisfensters in dem Referenzgenom basierend auf der Vielzahl der Bruchpunkte;

Bestimmen eines ersten Parameters basierend auf Reads, die in das Nachweisfenster fallen; und

Bestimmen der Existenz einer Kopiezahlvariante in der Genomprobe gegen das Nachweisfenster basierend auf einem Unterschied zwischen dem ersten Parameter und einem voreingestellten Schwellenwert;

wobei der Schritt des Bestimmens einer Vielzahl an Bruchpunkten in der Referenzgenomsequenz die Schritte umfasst:

Aufteilen der Referenzgenomsequenz in eine Vielzahl von primären Fenstern einer vorbestimmten Länge, und Bestimmen von Reads, die in jedes der Vielzahl von primären Fenstern fallen;

Bestimmen, für mindestens eine Stelle in der Referenzgenomsequenz, der Anzahl der Reads, die in die gleiche Anzahl der Vielzahl von primären Fenstern auf beiden Seiten der Stelle fallen; und

Bestimmen eines p-Werts der Stelle, wobei der p-Wert darstellt, dass die Anzahl der Reads, die in beide Seiten der Stelle fallen, Signifikanz hat; wobei die Stelle der Bruchpunkt ist, falls der p-Wert der Stelle kleiner ist als ein p-Endwert;

wobei der Schritt des Bestimmens des p-Werts der Stelle weiterhin umfasst:

Auswählen der Vielzahl der primären Fenster für die Stelle, die je die gleiche Anzahl auf beiden Seiten der Stelle haben, und Berechnen der relativen Zahl von Reads $R_i$, die in jedes der Vielzahl der primären Fenster fallen, wobei i die Anzahl der Vielzahl der primären Fenster darstellt, Unterziehen aller relativen Zahlen $R_i$ von Reads, die in die Vielzahl der primären Fenster fallen, einem Run-Test, um dadurch den p-Wert der Stelle zu bestimmen, wobei die relative Zahl von Reads durch die folgende Formel bestimmt wird:

$$R_i = \log_2\left(\frac{r_i}{\bar{r}}\right)$$

wobei $r_i$ die Anzahl der Reads, die in das i-te primäre Fenster fallen, darstellt,

$$\bar{r} = \frac{1}{n}\sum_{i=1}^{n} r_i \text{,}$$

n die Gesamtzahl der Vielzahl der primären Fenster darstellt.

2. Verfahren nach Anspruch 1, wobei das Verfahren weiterhin umfasst:

Lysieren der einzelnen Zelle, um das gesamte Genom der einzelnen Zelle freizusetzen; und Amplifizieren des gesamten Genoms, um die Genomprobe zu erhalten.

3. Verfahren nach Anspruch 1 oder 2, wobei die Kopiezahlvariante mindestens eine ausgewählt aus der Gruppe bestehend aus Aneuploidie eines Chromosoms, Deletion eines Chromosoms, Addition eines Chromosomfragments, Mikrodeletion eines Chromosomfragments und Mikrorepetition eines Chromosomfragments ist.

4. Verfahren nach einem der vorherigen Ansprüche, wobei die Reads, die in jedes der primären Fenster fallen, vorzugsweise individuell-alignierte Reads sind, wobei vorzugsweise 100 der primären Fenster von beiden Seiten der Stelle ausgewählt sind, wobei vorzugsweise die primären Fenster eine Länge von 100 Kbp bis 200 Kbp, noch bevorzugter 150 Kbp haben, wobei vorzugsweise der p-Endwert $1.1 \times 10^{-50}$ oder weniger beträgt.

5. Verfahren nach Anspruch 1, wobei das Unterziehen aller relativen Zahlen der Reads, die in die Vielzahl der primären Fenster fallen, einem Run-Test weiterhin umfasst:

Unterziehen der relativen Zahl von Reads $R_i$, die in jedes der Vielzahl der primären Fenster fallen, einer Korrektur des GC-Gehalts, um eine korrigierte relative Zahl von Reads $\tilde{R}_i$ zu erhalten; Bestimmen einer normalisierten Zahl von Reads $Z_i$, die in jedes der Vielzahl der primären Fenster fallen, basierend auf der korrigierten relativen Zahl von Reads; und Unterziehen aller normalisierten Zahlen $Z_i$ von Reads, die in jedes der Vielzahl der primären Fenster fallen, einem Run-Test.

6. Verfahren nach Anspruch 5, wobei die korrigierte relative Zahl an Reads $\tilde{R}_i$ durch die folgenden Schritte erhalten wird:

Berechnen eines GC-Gehalts von jedem der Vielzahl der primären Fenster; Aufteilen des GC-Gehalts in eine Vielzahl an Regionen in einer Einheit von 0.001, und Berechnen eines Durchschnittswerts $M_s$ von allen relativen Zahlen von Reads, die in jede der Vielzahl von Regionen fallen, wobei s die Anzahl der Vielzahl von Regionen darstellt; Bestimmen der korrigierten relativen Zahl von Reads $\tilde{R}_i$ basierend auf der folgenden Formel:

$$\tilde{R}_i = R_i - M_s \text{;}$$

und

Bestimmen der normalisierten Zahl von Reads $Z_i$ basierend auf der folgenden Formel: wobei

$$Z_i = \left( R_i - \tilde{R}_i - mean \right) / SD ,$$

wobei

$$mean = \frac{1}{n} \sum_{i=1}^{n} \left( R_i - \tilde{R}_i \right) ,$$

und

$$SD = \sqrt{ \frac{1}{n-1} \sum_{i=1}^{n} \left( R_i - \tilde{R}_i - mean \right)^2 } .$$

**7.** Verfahren nach einem der vorherigen Ansprüche, wobei der Schritt des Bestimmens eines Nachweisfensters in dem Referenzgenom basierend auf der Vielzahl der Bruchpunkte weiterhin umfasst:

1) Bestimmen einer Vielzahl von möglichen Bruchpunkten, wobei andere Bruchpunkte sowohl vor als auch nach den möglichen Bruchpunkten existieren;
2) Bestimmen eines p-Werts für jeden möglichen Bruchpunkt und Entfernen eines möglichen Bruchpunkts, der den p-Endwert hat;
3) Durchführen von Schritt 2) mit dem Rest der möglichen Bruchpunkte, bis alle p-Werte des Rests der möglichen Bruchpunkte alle kleiner als der p-Endwert sind, wobei der Rest der möglichen Bruchpunkte als gefilterte mögliche Bruchpunkte gewertet werden; und
4) Bestimmen einer Region zwischen zwei aufeinander folgenden gefilterten möglichen Bruchpunkten als das Nachweisfenster,

wobei der p-Wert des möglichen Bruchpunkts durch die folgenden Schritte erhalten wird:

Auswählen einer Region zwischen dem möglichen Bruchpunkt und einem vorherigen möglichen Bruchpunkt als eine erste mögliche Region, und Auswählen einer Region zwischen dem möglichen Bruchpunkt und einem folgenden möglichen Bruchpunkt als eine zweite mögliche Region;
Unterziehen der normalisierten Zahl $Z_i$ von Reads, die in die primären Fenster fallen, welche sowohl in der ersten möglichen Region als auch in der zweiten möglichen Region enthalten sind, einem Run-Test, um dadurch den p-Wert der möglichen Bruchpunkte zu ermitteln.

**8.** Verfahren nach Anspruch 7, wobei der Schritt des Bestimmens eines ersten Parameters basierend auf Reads, die in das Nachweisfenster fallen, weiterhin umfasst:

Bestimmen eines Durchschnittswerts $\overline{Z}$ von allen normalisierten Zahlen von Reads, die in jedes der Vielzahl der primären Fenster fallen, die in den Nachweisfenstern enthalten sind, wobei der Durchschnittswert $\overline{Z}$ der normalisierten Zahlen von Reads als der erste Parameter gewertet wird.

**9.** Verfahren nach Anspruch 1, wobei der voreingstellte Schwellenwert umfasst:

einen ersten Schwellenwert von -1.645 und einen zweiten Schwellenwert von 1.645.

**10.** Verfahren nach einem der vorherigen Ansprüche, wobei die Referenzgenomsequenz mindestens eine ausgewählt aus der Gruppe bestehend aus der Sequenz von menschlichem Chromosom 21, menschlichem Chromosom 18, menschlichem Chromosom 13, menschlichem Chromosom X, und menschlichem Chromosom Y ist.

**11.** System zur Bestimmung der Existenz einer Kopiezahlvariante in einer Genomprobe, umfassend:

ein Sequenzierungsapparat, dazu konfiguriert, die Genomprobe zu sequenzieren, um eine Sequenzierungsergebnis zu erhalten, das aus einer Vielzahl von Reads besteht, wobei die Genomprobe aus einer einzelnen Zelle gewonnen ist;

ein Analyseapparat, verbunden mit dem Sequenzierungsapparat und dazu konfiguriert zu bestimmen, ob eine Kopiezahlvariante in der Genomprobe besteht, basierend auf dem Sequenzierungsergebnis, wobei der Analyseapparat weiterhin umfasst:

eine Alignmenteinheit, dazu konfiguriert, das Sequenzierungsergebnis mit einer Referenzgenomsequenz zu alignieren, um eine Verteilung der Reads in der Referenzgenomsequenz zu bestimmen;

eine Bruchpunktbestimmungseinheit, verbunden mit der Alignmenteinheit und dazu konfiguriert, eine Vielzahl von Bruchpunkten in der Referenzgenomsequenz zu bestimmen, basierend auf der Verteilung der Reads in der Referenzgenomsequenz, wobei die Anzahl der Reads Signifikanz zwischen zwei Seiten der Bruchpunkte hat;

eine Nachweisfensterbestimmungseinheit, verbunden mit der Bruchpunktbestimmungseinheit und dazu konfiguriert, ein Nachweisfenster in dem Referenzgenom zu bestimmen, basierend auf der Vielzahl der Bruchpunkte;

eine Parameterbestimmungseinheit, verbunden mit der Nachweisfensterbestimmungseinheit und dazu konfiguriert, einen ersten Parameter zu bestimmen, basierend auf Reads, die in das Nachweisfenster fallen; und

eine Bestimmungseinheit, verbunden mit der Parameterbestimmungseinheit und dazu konfiguriert, die Existenz einer Kopiezahlvariante in der Genomprobe gegen das Nachweisfenster zu bestimmen, basierend auf einem Unterschied zwischen dem ersten Parameter und einem voreingestellten Schwellenwert,

wobei die Bruchpunktbestimmungseinheit konfiguriert ist

die Referenzgenomsequenz in eine Vielzahl von primären Fensters einer vorbestimmten Länge aufzuteilen, und Reads, die in jedes der Vielzahl von primären Fenstern fallen, zu bestimmen;

für mindestens eine Stelle in der Referenzgenomsequenz, die Anzahl der Reads, die in die gleiche Anzahl der Vielzahl von primären Fenstern auf beiden Seiten der Stelle fallen, zu bestimmen; und

einen p-Wert der Stelle zu bestimmen, wobei der p-Wert darstellt, dass die Anzahl der Reads, die in jegliche Seiten der Stelle fallen, Signifikanz haben; wobei die Stelle der Bruchpunkt ist, falls der p-Wert der Stelle kleiner ist als ein p-Endwert;

wobei der Schritt des Bestimmens des p-Werts der Stelle weiterhin umfasst:

Auswählen der Vielzahl der primären Fenster für die Stelle, die je die gleiche Anzahl auf beiden Seiten der Stelle haben, und Berechnen der relativen Zahl $R_i$ von Reads, die in jedes der Vielzahl der primären Fenster fallen, wobei i die Anzahl der Vielzahl der primären Fenster darstellt,

Unterziehen aller relativen Zahlen $R_i$ von Reads, die in die Vielzahl der primären Fenster fallen, einem Run-Test, um dadurch den p-Wert der Stelle zu bestimmen,

wobei die relative Zahl von Reads durch die folgende Formel bestimmt wird:

$$R_i = \log_2\left(\frac{r_i}{\overline{r}}\right)$$

wobei $r_i$ die Anzahl der Reads, die in das i-te primäre Fenster fallen, darstellt,

$$\overline{r} = \frac{1}{n}\sum_{i=1}^{n} r_i,$$

n die Gesamtzahl der Vielzahl der primären Fenster darstellt.

**12.** System nach Anspruch 11, weiterhin umfassend einen Genomextraktionsapparat, dazu konfiguriert, die Genomprobe aus einer biologischen Probe zu extrahieren.

**13.** System nach Anspruch 11 oder 12, wobei der Sequenzierungsapparat weiterhin umfasst:

eine Genomamplifikationseinheit, dazu konfiguriert die Genomprobe zu amplifizieren;
eine Sequenzierungsbibliotheksaufbaueinheit, verbunden mit der Genomamplifikationseinheit und dazu konfiguriert, eine Sequenzierungsbibliotehk mit der amplifizierten Genomprobe aufzubauen; und
eine Sequenzierungseinheit, verbunden mit der Sequenzierungsbibliotheksaufbaueinheit und dazu konfiguriert, die Sequenzierungsbibliothek zu sequenzieren.

14. Computerlesbares Medium, umfassend eine Anordnung, dazu konfiguriert, durch einen Prozessor auszuführen um die Existenz einer Kopiezahlvariante in einer Genomprobe durch die folgenden Schritte zu bestimmen:

Alignment eines Sequenzierungsergebnisses mit einer Referenzgenomsequenz, um eine Verteilung der Reads in der Referenzgenomsequenz zu bestimmen;
Bestimmen einer Vielzahl von Bruchpunkten in der Referenzgenomsequenz basierend auf der Verteilung der Reads in der Referenzgenomsequenz, wobei die Anzahl der Reads Signifikanz auf beiden Seiten der Bruchpunkte hat;
Bestimmen eines Nachweisfensters in dem Referenzgenom basierend auf der Vielzahl der Bruchpunkte;
Bestimmen eines ersten Parameters basierend auf Reads, die in das Nachweisfenster fallen; und
Bestimmen der Existenz einer Kopiezahlvariante in der Genomprobe gegen das Nachweisfenster basierend auf einem Unterschied zwischen dem ersten Parameter und einem voreingestellten Schwellenwert;
wobei optional der Schritt des Bestimmens einer Vielzahl an Bruchpunkten in der Referenzgenomsequenz die folgenden Schritte umfasst:

Aufteilen der Referenzgenomsequenz in eine Vielzahl von primären Fensters einer vorbestimmten Länge, und Bestimmen von Reads, die in jedes der Vielzahl von primären Fenstern fallen;
Bestimmen der Anzahl der Reads für mindestens eine Stelle in der Referenzgenomsequenz, die in die gleiche Anzahl der Vielzahl von primären Fenstern auf beiden Seiten der Stelle fallen; und
Bestimmen eines p-Werts der Stelle, wobei der p-Wert darstellt, dass die Anzahl der Reads, die auf beide Seiten der Stelle fallen, Signifikanz hat; wobei die Stelle der Bruchpunkt ist, falls der p-Wert der Stelle kleiner ist als ein p-Endwert;
wobei der Schritt des Bestimmens des p-Werts der Stelle weiterhin umfasst:

Auswählen der Vielzahl der primären Fenster für die Stelle, die je die gleiche Anzahl auf beiden Seiten der Stelle haben und Berechnen der relativen Zahl $R_i$ von Reads, die in jedes der Vielzahl der primären Fenster fallen, wobei i die Anzahl der Vielzahl der primären Fenster darstellt,
Unterziehen aller relativen Zahlen $R_i$ von Reads, die in die Vielzahl der primären Fenster fallen, einem Run-Test, um dadurch den p-Wert der Stelle zu bestimmen,
wobei die relative Zahl von Reads durch die folgende Formel bestimmt wird:

$$R_i = \log_2\left(\frac{r_i}{\bar{r}}\right)$$

wobei $r_i$ die Anzahl der Reads, die in das i-te primäre Fenster fallen, darstellt,

$$\bar{r} = \frac{1}{n}\sum_{i=1}^{n} r_i \, ,$$

n die Gesamtzahl der Vielzahl der primären Fenster darstellt.

**Revendications**

1. Procédé destiné à déterminer s'il existe une variation du nombre de copies dans un échantillon de génome, comprenant :

le fait de séquencer l'échantillon de génome, afin d'obtenir un résultat de séquençage constitué par une pluralité de relevés, sachant que l'échantillon de génome est obtenu à partir d'une cellule unique ;

le fait d'aligner le résultat de séquençage sur une séquence de génome de référence, afin de déterminer une distribution des relevés dans la séquence de génome de référence ;

le fait de déterminer une pluralité de points de rupture dans la séquence de génome de référence sur la base de la distribution des relevés dans la séquence de génome de référence, sachant que le nombre de relevés est significatif sur les deux côtés des points de rupture ;

le fait de déterminer une fenêtre de détection dans le génome de référence sur la base de la pluralité de points de rupture ;

le fait de déterminer un premier paramètre sur la base de relevés figurant dans la fenêtre de détection ; et

le fait de déterminer si la variation du nombre de copies existe dans l'échantillon de génome par rapport à la fenêtre de détection sur la base d'une différence entre le premier paramètre et un seuil prédéfini ;

sachant que l'étape consistant à déterminer une pluralité de points de rupture dans la séquence de génome de référence comprend les étapes consistant à

diviser la séquence de génome de référence en une pluralité de fenêtres primaires ayant une longueur prédéterminée, et déterminer des relevés figurant dans chacune de la pluralité des fenêtres primaires ;

pour au moins un site dans la séquence de génome de référence, déterminer le nombre de relevés figurant dans le même nombre de la pluralité de fenêtres primaires sur les deux côtés du site ; et

déterminer une valeur p du site, sachant que la valeur p représente le fait que le nombre de relevés figurant dans chacun des côtés du site est significatif ; sachant que le site est le point de rupture, si la valeur p du site est inférieure à une valeur p finale ;

sachant que l'étape consistant à déterminer la valeur p du site comprend en outre :

pour le site, le fait de sélectionner la pluralité de fenêtres primaires ayant le même nombre sur chacun des côtés du site respectivement, et le fait de calculer le nombre relatif de relevés figurant dans chacune de la pluralité de fenêtres primaires $R_i$, sachant que i représente le nombre de la pluralité de fenêtres primaires, le fait de soumettre la totalité des nombres relatifs de relevés figurant dans la pluralité de fenêtres primaires $R_i$ à un test d'exécution, pour déterminer de la sorte la valeur p du site,

sachant que le nombre relatif de relevés est déterminé par la formule suivante :

$$R_i = \log_2\left(\frac{r_i}{\overline{r}}\right)$$

où $r_i$ représente le nombre de relevés figurant dans la i-ème fenêtre primaire,

$$\overline{r} = \frac{1}{n}\sum_{i=1}^{n} r_i,$$

sachant que n représente le nombre total de la pluralité de fenêtres primaires.

2. Le procédé de la revendication 1, sachant que le procédé comprend en outre :

le fait de lyser la cellule unique, pour libérer tout le génome de la cellule unique ; et
le fait d'amplifier tout le génome pour obtenir l'échantillon de génome.

3. Le procédé de la revendication 1 ou 2, sachant que la variation du nombre de copies est au moins un élément sélectionné dans le groupe constitué par une aneuploïdie de chromosome, une suppression de chromosome, une addition d'un fragment de chromosome, une micro-suppression d'un fragment de chromosome et une micro-répétition d'un fragment de chromosome.

4. Le procédé de l'une quelconque des revendications précédentes, sachant que les relevés figurant dans chacune des fenêtres primaires sont de préférence des relevés alignés de façon unique,
de préférence, 100 des fenêtres primaires sont sélectionnées à partir de chacun des côtés du site,

de préférence, les fenêtres primaires ont une longueur de 100 Kbp à 200 Kbp, plus préférentiellement de 150 Kbp, de préférence, la valeur p finale est de $1,1 \times 10^{-50}$ ou moins.

5. Le procédé de la revendication 1, sachant que le fait de soumettre la totalité des nombres relatifs des relevés figurant dans chacune de la pluralité de fenêtres primaires à un test d'exécution comprend en outre :

le fait de soumettre le nombre relatif des relevés figurant dans chacune de la pluralité de fenêtres primaires $R_i$ à une correction de teneur en GC, pour obtenir un nombre relatif corrigé de relevés $\tilde{R}_i$ ;
le fait de déterminer un nombre normalisé de relevés figurant dans chacune de la pluralité de fenêtres primaires $Z_i$ sur la base du nombre relatif corrigé de relevés ; et
le fait de soumettre la totalité des nombres normalisés de relevés figurant dans chacune de la pluralité de fenêtres primaires $Z_i$ à un test d'exécution.

6. Le procédé de la revendication 5, sachant que le nombre relatif corrigé de relevés $\tilde{R}_i$ est obtenu par les étapes suivantes consistant à :

calculer une teneur en GC de chacune de la pluralité de fenêtres primaires ;
diviser la teneur en GC en une pluralité de régions dans une unité de 0,001, et
calculer une valeur moyenne $M_s$ parmi la totalité des nombres relatifs de relevés figurant dans chacune de la pluralité de régions, sachant que s représente le nombre de la pluralité de régions ;
déterminer le nombre relatif corrigé de relevés $\tilde{R}_i$ sur la base de la formule suivante :

$$\tilde{R}_i = R_i - M_s ;$$

et
déterminer le nombre normalisé de relevés $Z_i$ sur la base de la formule suivante :

$$Z_i = (R_i - \tilde{R}_i - moyenne)/SD ,$$

sachant que

$$moyenne = \frac{1}{n} \sum_{i=1}^{n}(R_i - \tilde{R}_i),$$

et

$$SD = \sqrt{\frac{1}{n-1} \sum_{i=1}^{n}(R_i - \tilde{R}_i - moyenne)^2}.$$

7. Le procédé de l'une quelconque des revendications précédentes, sachant que l'étape consistant à déterminer une fenêtre de détection dans le génome de référence sur la base de la pluralité des points de rupture comprend en outre :

1) le fait de déterminer une pluralité de points de rupture candidats, sachant qu'il existe d'autres points de rupture à la fois avant et après les points de rupture candidats ;
2) le fait de déterminer une valeur p de chaque point de rupture candidat, et le fait d'enlever un point de rupture candidat ayant la valeur p finale ;
3) le fait d'effectuer l'étape 2) avec le reste des points de rupture candidats jusqu'à ce que toutes les valeurs p du reste des points de rupture candidats soient toutes inférieures à la valeur p finale, sachant que le reste des points de rupture candidats est pris comme points de rupture candidats triés ; et
4) le fait de déterminer une région entre deux points de rupture candidats triés successifs comme étant la fenêtre de détection,

sachant que la valeur p du point de rupture candidat est obtenue par les étapes suivantes consistant à :

sélectionner une région entre le point de rupture candidat et un point de rupture candidat précédent comme étant une première région candidate, et sélectionner une région entre le point de rupture candidat et un point de rupture candidat suivant comme étant une deuxième région candidate ;

soumettre le nombre normalisé de relevés figurant dans les fenêtres primaires Zi qui sont incluses à la fois dans la première région candidate et la deuxième région candidate à un test d'exécution, pour déterminer ainsi la valeur p des points de rupture candidats.

8. Le procédé de la revendication 7, sachant que l'étape consistant à déterminer un premier paramètre sur la base des relevés figurant dans la fenêtre de détection comprend en outre :

le fait de déterminer une valeur moyenne parmi la totalité des nombres normalisés de relevés figurant dans chacune de la pluralité de fenêtres primaires $\overline{Z}$ qui sont incluses dans les fenêtres de détection, sachant que la valeur moyenne des nombres normalisés de relevés $\overline{Z}$ est prise comme étant le premier paramètre.

9. Le procédé de la revendication 1, sachant que le seuil prédéfini comprend :

un premier seuil qui est de -1,645 et un deuxième seuil qui est de 1,645.

10. Le procédé de l'une quelconque des revendications précédentes, sachant que la séquence de génome de référence est au moins une séquence sélectionnée dans le groupe constitué par la séquence de chromosome humain 21, chromosome humain 18, chromosome humain 13, chromosome humain X, et chromosome Y.

11. Système destiné à déterminer s'il existe une variation du nombre de copies dans un échantillon de génome, comprenant :

un appareil de séquençage, configuré pour séquencer l'échantillon de génome, afin d'obtenir un résultat de séquençage constitué par une pluralité de relevés, sachant que l'échantillon de génome est obtenu à partir d'une cellule unique ;

un appareil d'analyse, connecté à l'appareil de séquençage, et configuré pour déterminer s'il existe une variation du nombre de copies dans l'échantillon de génome sur la base du résultat de séquençage, sachant que l'appareil d'analyse comprend en outre :

une unité d'alignement, configurée pour aligner le résultat de séquençage sur une séquence de génome de référence, afin de déterminer une distribution des relevés dans la séquence de génome de référence ;

une unité de détermination de points de rupture, connectée à l'unité d'alignement, et configurée pour déterminer une pluralité de points de rupture dans la séquence de génome de référence sur la base de la distribution des relevés dans la séquence de génome de référence, sachant que le nombre de relevés est significatif entre deux côtés des points de rupture ;

une unité de détermination de fenêtre de détection, connectée à l'unité de détermination de points de rupture, et configurée pour déterminer une fenêtre de détection dans le génome de référence sur la base de la pluralité de points de rupture ;

une unité de détermination de paramètre, connectée à l'unité de détermination de fenêtre de détection, et configurée pour déterminer un premier paramètre sur la base de relevés figurant dans la fenêtre de détection ; et

une unité de détermination, connectée à l'unité de détermination de paramètre, et configurée pour déterminer si la variation du nombre de copies existe dans l'échantillon de génome par rapport à la fenêtre de détection sur la base d'une différence entre le premier paramètre et un seuil prédéfini,

sachant que l'unité de détermination de points de rupture est configurée

pour diviser la séquence de génome de référence en une pluralité de fenêtres primaires ayant une longueur prédéterminée, et pour déterminer des relevés figurant dans chacune de la pluralité de fenêtres primaires ;

pour déterminer, pour au moins un site dans la séquence de génome de référence, le nombre de relevés figurant dans le même nombre de la pluralité de fenêtres primaires sur les deux côtés du site ; et

pour déterminer une valeur p du site, sachant que la valeur p représente le fait que le nombre de relevés figurant dans chacun des côtés du site est significatif ; sachant que le site est le point de rupture, si la valeur p du site est inférieure à une valeur p finale ;

sachant que l'étape consistant à déterminer la valeur p du site comprend en outre :

pour le site, le fait de sélectionner la pluralité de fenêtres primaires ayant le même nombre sur chacun des côtés du site respectivement, et le fait de calculer le nombre relatif de relevés figurant dans chacune de la pluralité de fenêtres primaires $R_i$, sachant que i représente le nombre de la pluralité de fenêtres primaires,

le fait de soumettre la totalité des nombres relatifs de relevés figurant dans la pluralité de fenêtres primaires $R_i$ à un test d'exécution, pour déterminer ainsi la valeur p du site,

sachant que le nombre relatif de relevés est déterminé par la formule suivante :

$$R_i = \log_2\left(\frac{r_i}{\overline{r}}\right)$$

où $r_i$ représente le nombre de relevés figurant dans la i-ème fenêtre primaire,

$$\overline{r} = \frac{1}{n}\sum_{i=1}^{n} r_i \, ,$$

sachant que n représente le nombre total de la pluralité de fenêtres primaires.

12. Le système de la revendication 11, comprenant en outre un appareil d'extraction de génome, configuré pour extraire l'échantillon de génome à partir d'un échantillon biologique.

13. Le système de la revendication 11 ou 12, sachant que l'appareil de séquençage comprend en outre :

une unité d'amplification de génome, configurée pour amplifier l'échantillon de génome ;
une unité d'établissement de bibliothèque de séquençage, connectée à l'unité d'amplification de génome, et configurée pour construire une bibliothèque de séquençage avec échantillon de génome amplifié ; et
une unité de séquençage, connectée à l'unité d'établissement de bibliothèque de séquençage, et configurée pour séquencer la bibliothèque de séquençage.

14. Support lisible par ordinateur, comprenant un ordre, configuré pour être exécuté par un processeur afin de déterminer s'il existe une variation du nombre de copies dans un échantillon de génome via les étapes suivantes consistant à :

aligner un résultat de séquençage sur une séquence de génome de référence, afin de déterminer une distribution de relevés dans une séquence de génome de référence ;
déterminer une pluralité de points de rupture dans la séquence de génome de référence sur la base de la distribution des relevés dans la séquence de génome de référence, sachant que le nombre de relevés est significatif sur les deux côtés des points de rupture ;
déterminer une fenêtre de détection dans le génome de référence sur la base de la pluralité de points de rupture ;
déterminer un premier paramètre sur la base de relevés figurant dans la fenêtre de détection ; et
déterminer si la variation du nombre de copies existe dans l'échantillon de génome par rapport à la fenêtre de détection sur la base d'une différence entre le premier paramètre et un seuil prédéfini ;
sachant que facultativement l'étape consistant à déterminer une pluralité de points de rupture dans la séquence de génome de référence comprend en outre :

le fait de diviser la séquence de génome de référence en une pluralité de fenêtres primaires ayant une longueur prédéterminée, et déterminer des relevés figurant dans chacune de la pluralité de fenêtres primaires ;
pour au moins un site dans la séquence de génome de référence, le fait de déterminer le nombre de relevés figurant dans le même nombre de la pluralité de fenêtres primaires sur les deux côtés du site ;
le fait de déterminer une valeur p du site, sachant que la valeur p représente le fait que le nombre de relevés figurant dans chacun des côtés du site est significatif ; et
sachant que si la valeur p du site est inférieure à une valeur p finale, le site est les points de rupture ;
sachant que l'étape consistant à déterminer la valeur p du site comprend en outre :

pour le site, le fait de sélectionner la pluralité de fenêtres primaires ayant le même nombre sur chacun des côtés du site respectivement, et le fait de calculer le nombre relatif de relevés figurant dans chacune de la pluralité de fenêtres primaires $R_i$, sachant que i représente le nombre de la pluralité de fenêtres primaires,

le fait de soumettre la totalité des nombres relatifs de relevés figurant dans la pluralité de fenêtres primaires $R_i$ à un test d'exécution, pour déterminer ainsi la valeur p du site,

sachant que le nombre relatif de relevés est déterminé par la formule suivante :

$$R_i = \log_2\left(\frac{r_i}{\overline{r}}\right)$$

sachant que $r_i$ représente le nombre de relevés figurant dans la i-ème fenêtre primaire,

$$\overline{r} = \frac{1}{n}\sum_{i=1}^{n} r_i,$$

sachant que n représente le nombre total de la pluralité de fenêtres primaires.

s100

| sequencing the genome sample, to obtain a sequencing result consisting of a plurality of reads |

s200

| aligning the sequencing result to a reference genome sequence, to determine a distribution of the reads in the reference genome sequence |

s300

| determining a plurality of breakpoints in the reference genome sequence based on the distribution of the reads in the reference genome sequence |

s400

| etermining a first parameter based on reads falling in the detection window |

s500

| determining whether the copy number variation presents in the genome sample against the detection window based on difference between the first parameter and a preset threshold |

FIG.1

**1000**

FIG.2

```
┌─────────────────────────────┐         ┌─────────────────────┐
│ whole genome amplification  │◄────────│   testing sample    │
└─────────────────────────────┘         └─────────────────────┘
              │
              ▼
┌─────────────────────────────┐
│       sequencing data       │
└─────────────────────────────┘
              │
              ▼
┌─────────────────────────────┐
│       SOAP2 alignment       │
└─────────────────────────────┘
              │                         ┌─────────────────────┐
              │◄────────────────────────│   window selection  │
              ▼                         └─────────────────────┘
┌─────────────────────────────┐
│       basic statistics      │
└─────────────────────────────┘
              │
              ▼
┌─────────────────────────────┐
│     correction of GC bias   │
└─────────────────────────────┘
              │
              ▼
┌─────────────────────────────┐
│   Statistical test of       │
│   detecting signal          │
│   breakpoint                │
└─────────────────────────────┘
              │
              ▼
┌─────────────────────────────┐
│   connecting windows and    │
│      filtering singles      │
└─────────────────────────────┘
```

Fig.3

Fig.4-1

Repeat
Deletion
Normal Region

Fig.4-2

38

Fig.5-1

Repeat
Deletion
Normal Region

EP 2 826 865 B1

Fig.5-2

**EP 2 826 865 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **YOON et al.** *Genome Research,* 2009, vol. 19 (9), 1586-1592
- *The Annals of Mathematical Statistics,* 1940, vol. 11, 147-162

- **J. SAMBROOK et al.** Molecular Cloning: A Laboratory Manual. Science Press